# EUROPEAN PATENT SPECIFICATION

(11) **EP 4 460 269 B1**
(45) Date of publication and mention of the grant of the patent: **08.04.2026**
(21) Application number: 23729820.3
(22) Date of filing: 15.05.2023
(51) Int. Cl.: A61F 5/01, A61H 3/00, A63B 69/18, A63C 11/00

(54) **BODY WEIGHT SUPPORT**
KÖRPERGEWICHTSSTÜTZE
SUPPORT DE POIDS CORPOREL

(30) Priority: 17.05.2022 GB 202207217
(43) Date of publication of application: 13.11.2024
(73) Proprietor: Mojo Technologies SAS, 74230 Thones (FR)
(72) Inventor: HANNAFORD, Martin, Horsham West Sussex RH12 4SP (GB)
(74) Representative: Gillott-Jones, Nathan Philip
(86) International application number: PCT/GB2023/051268
(87) International publication number: WO 2023/223005

(56) References cited:
- EP-A2- 3 666 242
- GB-A- 2 260 083
- US-A1- 2016 158 087

## Description

### FIELD

The present disclosure relates to a body weight support. In particular, the present disclosure relates to a body weight support for supporting a user's body weight during an activity such as, but not limited to, skiing.

### BACKGROUND

Skiing is a sport that can result in significant loads on the muscles and joints of the skier, particularly in the legs. The skier must repeatedly bend the legs, whilst at the same time the legs must continue to support the skier. The loads are often increased as a result of the skier adopting an incorrect stance or posture, or from repeated impacts of the skis with uneven compacted snow or ice, causing a bouncing effect. Consequently, all skiers can benefit from the use of body weight supports that assist in setting the skier in a correct position and from reducing the forces on the skier's leg muscles and joints.

Body weight supports can also be used in other settings to support a user's body weight and reduce the load on the user's muscles and joints. For example, body weight supports can be used to support a user's weight during manual operations such as fruit picking and assembly line work, or any other activity involving a squatting motion.

Existing body weight supports include an upper member configured for attachment to a user's upper leg (e.g. around the thigh), a lower member configured for attachment to a user's lower leg (e.g. by attachment to a boot worn on the user's foot), and a pivotal connection between the upper and lower members. Such existing body weight supports also include a load bearing element that resists the pivotal movement between the upper and lower members. The load bearing element reduces the load on the user's muscles and joints when the user bends their legs, thereby supporting the user in a squat position.

The upper member of existing body weight supports is configured for location against the side of the user's outer thigh, so that the pivotal connection between the upper and lower members is alongside the user's natural pivot point (i.e. the user's knee). The lower member of existing body weight supports is configured for attachment to the rear of the user's boot, in a substantially central position. Attachment of the body weight support to the rear of the user's boot prevents a twisting or turning force from being applied to the user's boot, which would occur, for example, if the lower member were attached to a side of the boot. Accordingly, such an attachment prevents the user's foot (and consequently the user's skis) from being turned inwards.

In order to accommodate the positioning of the upper member against the side of the user's outer thigh and the attachment of the lower member to the rear of the user's boot, existing body supports such as those described in EP 1066808B1 and available from Ski-Mojo Limited of Horsham, UK include a curved lower member. The curved lower member also provides a more ergonomic fit, by accommodating the user's calf muscles, which generally are not in line with the upper thigh and protrude laterally outwards. In particular, the lower member incorporates a curved section between the pivot and the point of attachment to the user's boot. A drawback of such solutions is that the curved lower member limits the angular rotation about the pivot, because the curved lower member is forced into contact with the upper member at acute angles. Such situations may occur, for example, if the user squats deeply, or during folding of the support when not in use. By forcing the lower member into contact with the upper member, the upper member and/or lower member can become damaged. A further drawback is that the support cannot be compactly collapsed when not in use, as the curved lower member protrudes outwardly.

Other existing body weight supports, such as those available from Againer of Riga, Latvia include an upper member in the form of an upper shell that houses the user's upper leg, and a lower member in the form of a lower shell that houses the user's lower leg. The upper and lower shell are pivotally coupled using pivots that are located, in use, on either side of the user's knee. A gas strut is connected between the upper shell and the lower shell, to resist the pivotal movement between the upper and lower shells. It will be appreciated that such body weight supports also fail to accommodate deep squatting or folding movements, because the piston can only be moved so far into the cylinder of the gas strut. The range of movement of the piston relative to the cylinder also prevents compact collapsing of the support when not in use.

Accordingly, there exists a need for an improved body weight support that addresses the drawbacks identified above.

US 2016/158087 A1 describes a portable human exoskeleton system that includes a pelvis module, a leg module and a foot module. The pelvis module includes a bendable member and a pelvis module connector. The foot module includes a foot module connector. The leg module includes: a femur module detachably coupled to the pelvis module connector; a tibia module detachably coupled to the foot module connector; and a knee joint component having at least two linkages with different lengths wherein each linkage is pivotally coupled to the femur module and the tibia module. Weight above the hip of the user is exerted on the pelvis module, and transferred to the leg module and the foot module.

GB 2260083 A describes a caliper that comprises at least two pivotally-connected mutually-lockable "steels" for attachment to respective limb members. In order to provide sufficient flexion in the "steels" when unlocked and to prevent excessive loadings being placed on the lock components, the pivotal connection between the steels may be held in a specific locked position by a releasable lever in the form of a hook having an angled engagement to take up play in the linkage formed by the locking links. In particular, the locking lever is pivotally mounted on the link and has a slot or recess to receive the pivot pin between links.

EP 3666242 A2 describes a motion assistance apparatus that includes a proximal frame configured to support a proximal part of a user, a connecting frame rotatably connected to the proximal frame, a distal frame rotatably connected to the connecting frame and configured to support a distal part of the user, and a power transmitter including a slider configured to slide along the proximal frame, a pusher rotatably connected to the distal frame and the slider, and a coupler configured to connect the slider and the pusher and positioned closer to the proximal part of the user than a rotation axis of the connecting frame in the whole range of motion.

### SUMMARY

This summary introduces concepts that are described in more detail in the detailed description. It should not be used to identify essential features of the claimed subject matter, nor to limit the scope of the claimed subject matter.

In one aspect, there is provided a body weight support as defined in claim 1. Features of embodiments are defined in the dependent claims.

According to one example of the present disclosure, there is provided a body weight support, comprising: a first member configured for location against a first portion of a limb of a user, the first portion of the limb being on a first side of a joint of the user; a second member configured for location against a second portion of the limb on a second side of the joint of the user, wherein the second member is rotatable relative to the first member about a pivot, the second member comprising: a first portion proximate to the pivot; and a second portion extending from the first portion, distal to the pivot; wherein the second portion is movable from a first configuration in which a first angle between the first portion and the first member is less than a second angle between the second portion and the first member, to a second configuration in which the difference between the first angle and the second angle is less than the difference between the first angle and the second angle in the first configuration; and a load bearing element configured to provide a resistance to rotation of the second member relative to the first member.

By allowing the difference between the first angle and the second angle to be reduced (i.e. in the second configuration of the second portion), the second portion of the second member can be moved to a position in which the angle between the second portion and the first member is small. In an example in which the first member is configured for location against an upper leg and the second member is configured for location against a lower leg, the reduction in angle between the second portion of the second member and the first member means that the body weight support can support the user at acute angles between the first member and second member, such as the angles resulting from deep squat positions. Moreover, the reduction in angle between the second portion of the second member and the first member means that the body weight support can be compactly stored when not in use.

The second member may be rotatable from a first angular position relative to the first member to a second angular position relative to the first member, wherein the second portion is not movable from the first configuration to the second configuration when the second member is in the first angular position, and wherein the second portion is movable from the first configuration to the second configuration when the second member is in the second angular position. The body weight support therefore implements a "delayed hinge", where the first configuration of the second portion is maintained until the second member is at a particular angular position relative to the first member.

The second member may be rotatable from a first angular position relative to the first member to a second angular position relative to the first member, wherein rotation of the second portion of the second member relative to the first member does not cause movement of the second portion from the first configuration to the second configuration when the second member is in the first angular position, and wherein rotation of the second portion of the second member relative to the first member causes movement of the second portion from the first configuration to the second configuration when the second member is in the second angular position. Such an arrangement also implements the "delayed hinge" described above.

The second member may be rotatable from a first angular position relative to the first member to a second angular position relative to the first member, wherein rotation of the second member from the first angular position to the second angular position causes movement of the second portion from the first configuration to the second configuration. The second portion therefore moves constantly relative to the first portion as the second member is rotated relative to the first member.

When the second member is in the second angular position, an angle between the first portion and the first member may be smaller than when the second member is in the first angular position. In particular, when the second member is in the second angular position, the first portion may be substantially parallel to the first member.

When the second portion is in the second configuration, the second portion may be in line with the first portion. By providing a configuration in which the second portion is in line with the first portion, the user can be supported in deep squat positions and the body weight support can be compactly stored when not in use.

The second portion may be hingedly coupled to the first portion. The second portion may be prevented from hinging relative to the first portion until the second member is in the second angular position. Alternatively, the rotation of the second member relative to the first member may cause hinging of the second portion relative to the first portion.

The length of the second portion may be adjustable. Adjustment of the length of the second portion accommodates different lower limb lengths (e.g. different lower leg lengths). The length of the first portion may be adjustable. Adjustment of the length of the first portion accommodates different lower limb thicknesses (e.g. different lower leg thicknesses). An angle between the first portion and the second portion in the first configuration of the second member may be adjustable. The adjustment of the angle allows the body weight support to be adapted to the user's limbs, to optimise comfort.

The body weight support may further comprise a pivotal coupling configured to permit the rotation of the second member relative to the first member. The body weight support may further comprise a lever member coupled to the second member, wherein the lever member is configured to apply a frictional force to the pivotal coupling during rotation of the second member from the first angular position to the second angular position. The application of the frictional force to the pivotal coupling supplements the resistance provided by the load bearing element, to further resist rotation of the second member relative to the first member and thereby support the user's body weight.

The load bearing element may be deactivatable to permit substantially free rotation of the second member relative to the first member. Deactivation of the load bearing element allows the resistance provided by the load bearing element to be removed to allow for full leg mobility, thereby permitting normal sitting and walking movements while wearing the body weight support.

The first member may be configured for location against an upper leg of the user and the second member may be configured for location against a lower leg of the user. The first member may be configured for location against the outer side of the user's upper leg. This allows the pivot to be located adjacent to the user's knee, which is a natural pivot point. Accordingly, the second member pivots relative to the first member as the user's lower leg pivots relative to the user's upper leg.

The second portion may be connectable to a boot of the user. In particular, the second portion may be connectable to the rear of the boot of the user. Attachment of the body weight support to the rear of the user's boot prevents a twisting or turning force from being applied to the user's boot.

The body weight support may further comprise a connection member pivotally coupled to the first portion of the second member, wherein the connection member engages an end of the load bearing element. The length of the connection member may be adjustable. Adjustment of the length of the connection member allows the resistance provided by the load bearing element to be adjusted (e.g. depending on the user's weight). An end of the connection member may be slidably movable within a slot in the first member, and the end of the connection member may engage the end of the load bearing element.

The load bearing element may comprise a cable. Use of a cable allows a simpler mechanism to be provided to couple the second portion of the second member to the first member. In addition, the use of a cable reduces the force applied to a pivotal coupling that permits the rotation of the second member relative to the first member. Specifically, the force applied to the pivotal coupling is reduced when a first end of the cable is attached to the second portion of the second member.

### BRIEF DESCRIPTION OF FIGURES

Specific embodiments are described below by way of example only and with reference to the accompanying drawings, in which:
FIG. 1 shows a front view of a body weight support according to a first embodiment, in which a lower member of the body weight support is shown at a first angular position with respect to an upper member of the body weight support.
FIG. 2 shows a simplified rear view of a body weight support shown in FIG. 1, in which the lower member is shown at the first angular position.
FIGS. 3A to 3C show front views of the body weight support shown in FIG. 1, in which the lower member is shown at various angular positions with respect to the upper member.
FIGS. 4A to 4C show simplified rear views of the body weight support shown in FIG. 1, in which the lower member is shown at various angular positions corresponding to the angular positions shown in FIGS. 3A to 3C.
FIGS. 5A to 5D show front views of the body weight support shown in FIG. 1, in which the lower member is shown at various angular positions with respect to the upper member, and in which a toggle clip of the body weight support is in an open position.
FIGS. 6A to 6D show simplified rear views of a body weight support according to a second embodiment, in which a lower member of the body weight support is shown at various angular positions with respect to an upper member of the body weight support.
FIGS. 7A to 7E show simplified rear views of a body weight support according to a third embodiment, in which a lower member of the body weight support is shown at various angular positions with respect to an upper member of the body weight support.
FIGS. 8A to 8J show schematic rear views of a body weight support according to a fourth embodiment, in which a lower member of the body weight support is shown at various angular positions with respect to an upper member of the body weight support.
FIGS. 9A to 9F show schematic rear views of a body weight support according to a fifth embodiment, in which a lower member of the body weight support is shown at various angular positions with respect to an upper member of the body weight support.

### DETAILED DESCRIPTION

Implementations of the present disclosure are explained below with particular reference to body weight supports that reduce the load on a user's leg muscles and joints. It will be appreciated, however, that the devices described herein may also be applied to body weight supports that reduce the load on other limbs (e.g. arms). Moreover, implementations of the present disclosure are explained below with particular reference to skiing. It will further be appreciated that the devices described herein are also suitable for other activities in which a user's body weight is to be supported in order to reduce the load on the user's muscles and joints.

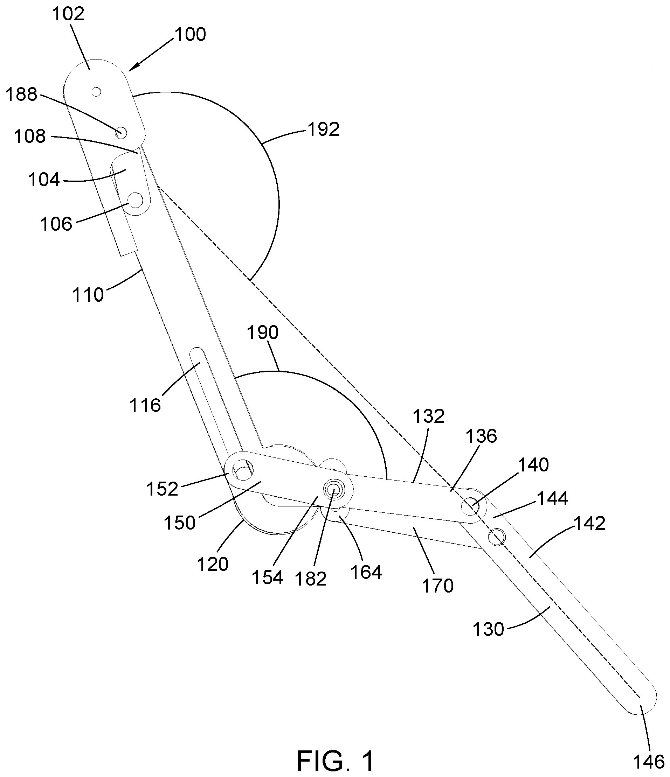

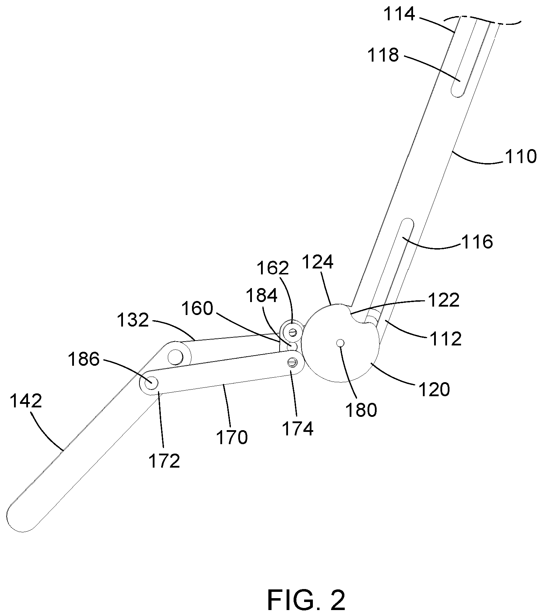

FIG. 1 is a schematic diagram of a front view of a body weight support 100 according to a first embodiment, while FIG. 2 is a schematic diagram of a rear view of the body weight support 100. FIG. 2 is a simplified view of the body weight support 100 shown in FIG. 1, because the toggle clip 102 (described below) is not shown in FIG. 2. The body weight support 100 comprises a first member in the form of an upper member 110 configured for location against an upper limb (e.g. an upper leg) of a user. The upper limb may be considered a first portion of a limb (e.g. a leg), which is on a first side of a joint (e.g. a knee) of the user. As one example, the upper member 110 may be secured against the outer side of the user's thigh using one or more straps (not shown in FIG. 1). The upper member 110 comprises a first end 112 (best shown in FIG. 2) that is fixedly coupled to a cam 120, and a second end 114 opposite the first end 112. The upper member 110 further comprises a first elongate slot in the form of a lower slot 116 extending along a portion of the upper member 110 towards the first end 112, and a second elongate slot in the form of an upper slot 118 (best shown in FIG. 2) extending along a portion of the upper member 110 towards the second end 114.

As shown in FIG. 2, the cam 120 to which the first end 112 of the upper member 110 is coupled comprises an indent 122 located adjacent to the point at which the cam 120 is coupled to the first end 112. Aside from the indent 122, the cam 120 comprises a surface 124 having a substantially constant radius.

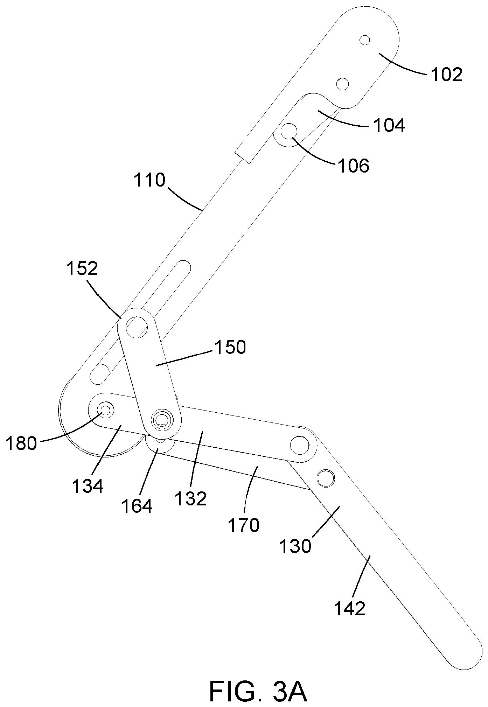

Returning to FIG. 1, the body weight support 100 further comprises a hinged second member in the form of a lower member 130. The lower member 130 is configured for location against a lower limb (e.g. a lower leg) of the user. The lower limb may be considered a second portion of the limb (e.g. leg), which is on a second side of the user's joint. The lower member 130 comprises a first portion 132 having a first end 134 pivotally coupled to the centre of the cam 120 at a first pivot 180 (as best shown in FIG. 3A), meaning that the first portion 132 is proximate to the first pivot 180. The pivotal coupling between the first end 134 of the first portion 132 and the cam 120 allows the first portion 132 (and therefore the lower member 130) to be rotated relative to the upper member 110. Returning to FIG. 1, it can be seen that the first portion 132 of the lower member 130 further comprises a second end 136 coupled to a hinge 140.

The lower member 130 also comprises a second portion 142 having a first end 144 coupled to the hinge 140, such that the second portion 142 extends distally from the first portion 132 and is distal to the first pivot 180. The second portion 142 also has a second end 146 at its distal end. In one example, the second end 146 is configured for connection to a rear portion of a boot (not shown) worn by the user.

The lower member 130 is configured for location against the lower limb of the user such that the second portion 142 is substantially parallel to the user's lower limb. The first portion 132 sits against the user's lower limb and connects the second portion 142 to the cam 120. In an example in which the body weight support 100 is worn on the user's leg, the second portion 142 is located against the rear of the user's lower leg, while the first portion 132 is located against the side of the user's lower leg, and extends between the second portion 142 and the cam 120 (which is adjacent to the user's knee joint). In one example, the length of the second portion 142 is adjustable (e.g. using a telescopic adjustment), in order to accommodate different lower limb lengths. In a further example, the length of the first portion 132 is adjustable (e.g. using a telescopic adjustment), in order to accommodate different lower limb thicknesses. In a further example, the angle between the first portion 132 and the second portion 142 is adjustable. For example, the second portion 142 may include a plurality of holes via which the first portion 132 may be hingedly attached to the second portion 142. Each of the plurality of holes may be located a different distance from the second end 146 of the second portion 142, thereby each allowing distinct connection angles between the first portion 132 and the second portion 142.

The body weight support 100 further comprises a connection member 150 having a first end 152 that is slidably moveable within the lower slot 116 in the upper member 110. A linear bearing (not shown) permits the first end 152 of the connection member 150 to slide within the lower slot 116. A second end 154 of the connection member 150 is pivotally coupled to the first portion 132 of the lower member 130 at a second pivot 182. The second pivot 182 allows the first end 152 of the connection member 150 to slide within the lower slot 116 as the lower member 130 is rotated relative to the upper member 110. In the example shown in FIG. 1, an anticlockwise rotation of the lower member 130 relative to the upper member 110 (i.e. towards the upper member 110) causes the first end 152 of the connection member 150 to be slidably displaced away from the cam 120 (as shown, for example, in FIG. 3A), whereas a clockwise rotation of the lower member 130 relative to the upper member 110 (i.e. away from the upper member 110) causes the first end 152 of the connection member 150 to be slidably displaced towards the cam 120 (e.g. to the position shown in FIG. 1).

As shown in FIG. 2, the body weight support 100 further comprises a bogie 160 pivotally coupled to the first portion 132 of the lower member 130 at a third pivot 184 located between a first end 162 of the bogie 160 and a second end 164 (best shown in FIG. 1) of the bogie 160. In the configuration shown in FIGS. 1 and 2, each of the first end 162 and the second end 164 contacts the substantially constant radius surface 124 of the cam 120, with the first end 162 being disposed closer to the indent 122 than the second end 164, as shown in FIG. 2. Each end 162, 164 comprises one or more bearings allowing the end 162, 164 to move against the substantially constant radius surface 124 of the cam 120.

A lever member in the form of a bogie lever 170 extends between the second portion 142 of the lower member 130 and the second end 164 of the bogie 160. The bogie lever 170 has a first end 172 that is pivotally coupled to the second portion 142 of the lower member 130 at a fourth pivot 186 located between the first end 144 and the second end 146 of the second portion 142. A second end 174 of the bogie lever 170 is pivotally coupled to the second end 164 of the bogie 160. The pivotal connections of the bogie lever 170 mean that, in the configuration shown in FIG. 2, a clockwise rotation of the second portion 142 of the lower member 130 urges the bogie 160 to rotate clockwise about the third pivot 184, because the bogie lever 170 is under tension when the second portion 142 is rotated clockwise.

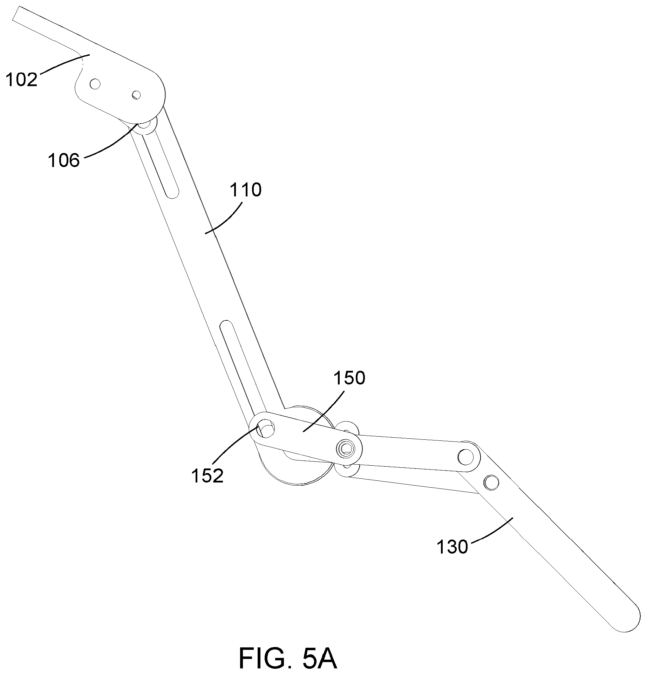

As shown in FIG. 1, the body weight support 100 further comprises a toggle clip 102 that is pivotally coupled to the first end 114 of the upper member 110. The toggle clip 102 is shown in a closed position in FIG. 1. The body weight support 100 also comprises a spring engagement member 104 having a first end 106 that is slidably movable within the upper slot 118 in the upper member 110. A linear bearing (not shown) permits the first end 106 of the spring engagement member 104 to slide within the upper slot 118. A second end 108 of the spring engagement member 104 is pivotally coupled to the toggle clip 102 at a fifth pivot 188. The fifth pivot 188 allows the first end 106 of the spring engagement member 104 to slide within the upper slot 118 as the toggle clip 102 is rotated from the closed position shown in FIG. 1 to the open position shown in FIGS. 5A to 5D. In the example shown in FIG. 1, a clockwise rotation of the toggle clip 102 about the pivotal coupling at the first end 114 of the upper member 110 causes the first end 106 of the spring engagement member 104 to be slidably displaced away from the cam 120 (as shown, for example, in FIG. 5A), while an anticlockwise rotation of the toggle clip 102 about the pivotal coupling at the first end 114 of the upper member 110 causes the first end 106 of the spring engagement member 104 to be slidably displaced towards the cam 120 (e.g. to the position shown in FIG. 1).

A load bearing element (not shown) is coupled between the first end 106 of the spring engagement member 104 and the first end 152 of the connection member 150. In one example, the load bearing element is coupled to the first end 152 of the connection member 150, and is not fixedly coupled to the first end 106 of the spring engagement member 104, such that the first end 106 of the spring engagement member 104 can be pulled away from the load bearing element when the toggle clip 102 is moved to the open position. It will be appreciated, however, that the load bearing element may instead be fixedly coupled to the first end 106 of the spring engagement member 104 and not fixedly coupled to the first end 152 of the connection member 150. As a further alternative, the load bearing element may not be fixedly coupled to the first end 106 of the spring engagement member 104 or to the first end 152 of the connection member 150, in which case the load bearing element may be held in a guide (e.g. a tube) between the first end 152 and the first end 106, and engaged by the first end 152 and the first end 106 when the toggle clip 102 is moved to the closed position.

The load bearing element provides a resistance to rotation of the lower member 130 relative to the upper member 110. In one example, the load bearing element is a spring that is compressed as the first end 152 of the connection member 150 moves towards the first end 106 of the spring engagement member 104. The compression of the spring causes the spring to exert a force on the first end 152 of the connection member 150 to bias the first end 152 of the connection member 150 towards the cam 120, thereby providing a resistance to rotation of the lower member 130 relative to the upper member 110. Specifically, in the example shown in FIG. 1, the spring provides a resistance to anticlockwise rotation of the lower member 130 relative to the upper member 110. It will be appreciated that, in other examples, other types of load bearing element (e.g. one or more gas struts) may be implemented instead of a spring. In one example, the length of the connection member 150 is adjustable, in order to provide adjustable levels of support resulting from adjustment of the force exerted by the load bearing element when compressed. Accordingly, the force exerted by the load bearing element may be adjusted depending on the user's weight.

When the body weight support 100 is in the configuration shown in FIGS. 1 and 2, the lower member 130 is in a first angular position relative to the upper member 110. In the first angular position, the second portion 142 of the lower member 130 is in a first configuration relative to the first portion 132 of the lower member 130. Specifically, the second portion 142 of the lower member 130 cannot be rotated relative to the first portion 132. This is because the contact between the first and second ends 162, 164 of the bogie 160 and the substantially constant radius surface 124 of the cam 120 prevents rotation of the bogie 160 about the third pivot 184 from being imparted via the bogie lever 170 by rotation of the second portion 142 either towards the upper member 110 or away from the upper member 110. Instead, the contact between the ends 162, 164 of the bogie and the substantially constant radius surface 124 results in a frictional force being applied to the cam 120 via the bogie lever 170 when the second portion 142 is rotated. The friction applied to the cam 120 provides additional resistance to rotation of the lower member 130 relative to the upper member 110.

As shown in FIG. 1, when the second portion 142 of the lower member 130 is in the first configuration relative to the first portion 132 of the lower member, the angle 190 between the first portion 132 and the upper member 110 is less than the angle 192 between the second portion 142 and the upper member 110.

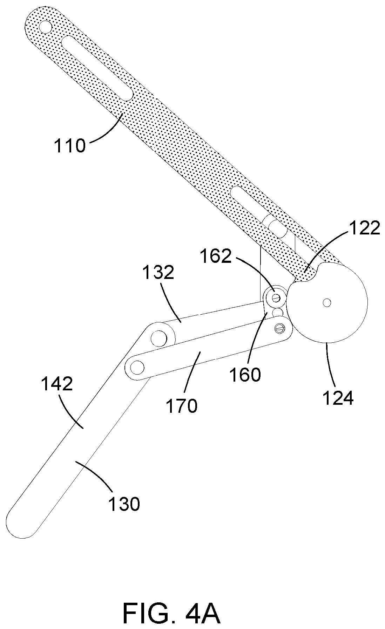

FIGS. 3A to 3C and 4A to 4C show rotation of the lower member 130 to various angular positions relative to the upper member 110. As with FIG. 2, FIGS. 4A to 4C show simplified views in which the toggle clip 102 and spring engagement member 104 are omitted. As shown in FIGS. 3A and 4A, further rotation of the lower member 130 relative to the upper member 110 results in the ends 162, 164 of the bogie 160 traversing the substantially constant radius surface 124 such that the first end 162 of the bogie 160 approaches the indent 122. FIGS. 3A and 4A also show that the rotation of the lower member 130 causes the first end 152 of the connection member 150 to be displaced away from the cam 120. This means that the spring (or other load bearing element) is compressed between the first end 152 of the connection member 150 and the first end 106 of the spring engagement member 104. The compression of the spring provides a resistance to the rotation of the lower member 130 towards the upper member 110.

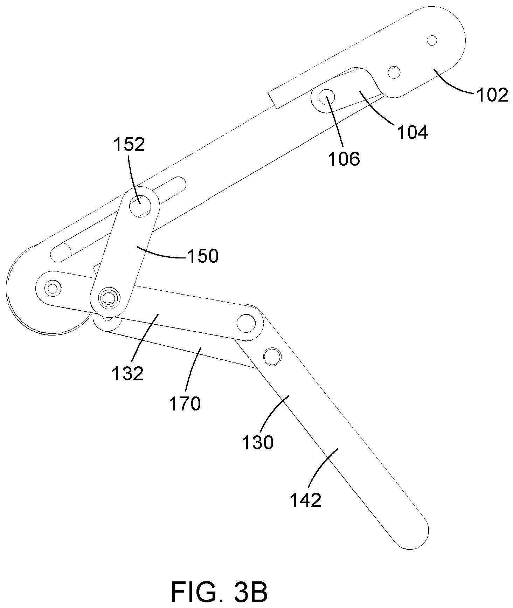

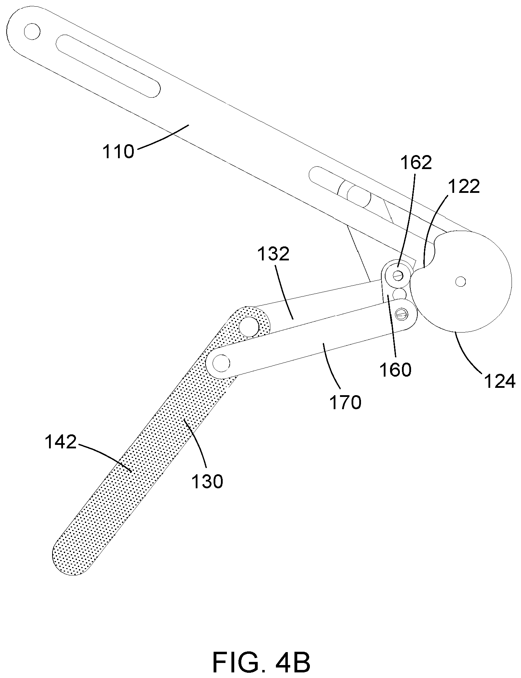

FIGS. 3B and 4B show the position of the lower member 130 when the first end 162 of the bogie 160 is about to move into the indent 122. In this angular position, the second portion 142 of the lower member 130 is still in the first configuration. The rotation of the lower member 130 to the position shown in FIGS. 3B and 4B causes the first end 152 of the connection member 150 to be displaced yet further from the cam 120, further compressing the spring such that the resistance to rotation of the lower member 130 towards the upper member 110 is increased when compared to the angular position shown in FIGS. 3A and 4A.

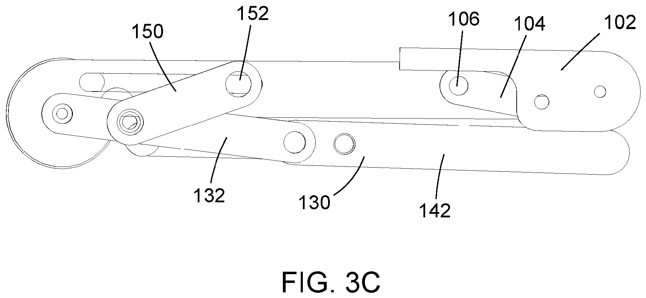

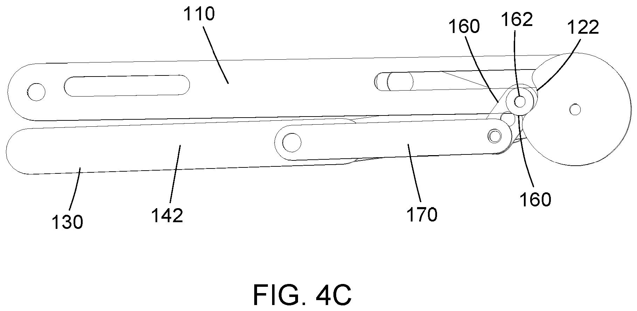

As shown in FIGS. 3C and 4C, once the first end 162 of the bogie 160 reaches the indent 122, the lower member 130 is in a second angular position relative to the first member 110. In particular, when the lower member 130 is in the second angular position, the angle 190 between the first portion 132 and the upper member 110 is less than when the lower member 130 is in the first angular position. Specifically, the first portion 132 is substantially parallel to the upper member 110 (such that the angle 190 between the first portion 132 and the upper member 110 is close to zero degrees). At the second angular position, the first end 162 of the bogie 160 can move in a radially inward direction, towards the first pivot 180. This means that the bogie 160 can rotate about the third pivot 184.

The rotation of the bogie 160 permits the second portion 142 of the first member 130 to be moved out of the first configuration shown in FIGS. 3B and 4B. In particular, the second portion 142 can be rotated relative to the first portion 132 about the hinge 140, so that the second portion 142 can be moved to a second configuration relative to the first portion 132. In the example shown in FIGS. 3C and 4C, when the second portion 142 is in the second configuration, the second portion 142 is aligned with (i.e. in line with) the first portion 132.

It will be appreciated that when the second portion 142 is in the second configuration relative to the first portion 132, the difference between the angle 190 and the angle 192 (close to zero in the example shown in FIGS. 3C and 4C) is less than the difference between the angle 190 and the angle 192 when the second portion 142 is in the first configuration relative to the first portion 132.

The rotation of the lower member 130 to the position shown in FIGS. 3C and 4C causes the first end 152 of the connection member 150 to be displaced even further from the cam 120, further compressing the spring such that the resistance to rotation of the lower member 130 towards the upper member 110 is increased when compared to the angular position shown in FIGS. 3B and 4B.

FIGS. 5A to 5D show rotation of the lower member 130 to angular positions that correspond to the angular positions shown in FIG. 1 and FIGS. 3A to 3C, but with the toggle clip 102 in an open position. As explained above, the load bearing element (e.g. a spring) is coupled to the first end 152 of the connection member 150, but is not fixedly coupled to the first end 106 of the spring engagement member 104. Accordingly, when the lower member 130 is in the first angular position relative to the upper member 110, shown in FIG. 5A, the first end 106 of the spring engagement member 104 is free of the end of the spring.

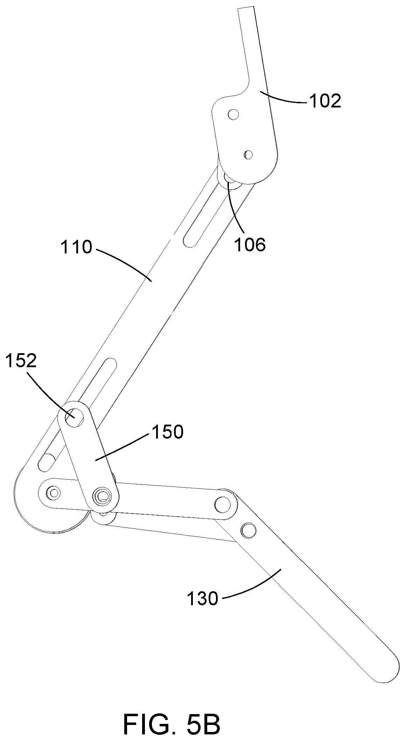

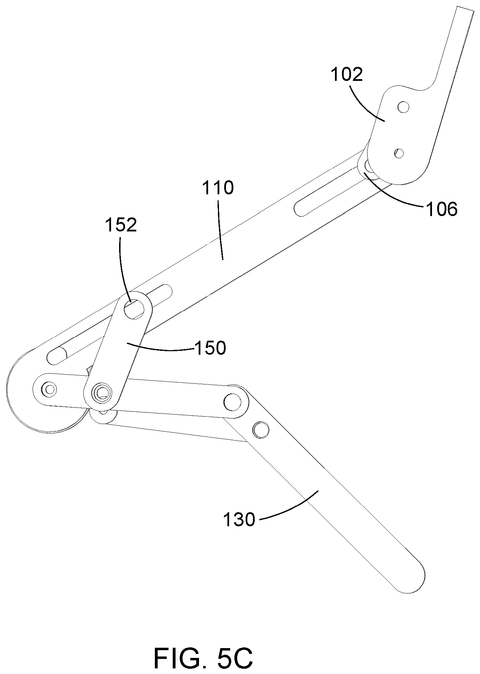

In one example, the length of the spring is approximately equal to the distance between the first end 106 of the spring engagement member 104 and the first end 152 of the connection member 150 when the toggle clip 102 is in the closed position and when the lower member 130 is in the first angular position relative to the upper member 110 (e.g. as shown in FIG. 1). This means that when the toggle clip 102 is in the open position and the lower member 130 is rotated towards the upper member 110 (e.g. as shown in FIGS. 5B and 5C), the spring is not compressed. This is because the distance between the first end 152 and the first end 106 in the configurations shown in FIGS. 5B and 5C exceeds the distance between the first end 152 and the first end 106 in the configuration shown in FIG. 1. Accordingly, the user can deactivate the load bearing element so that they can adopt a seated or squatting position without substantial resistance to rotation of the lower member 130 (i.e. such that there is substantially free rotation of the second member 130 relative to the first member 110). In this context, the term "substantially free rotation" is intended to convey that there will be a small amount of resistance to rotation resulting from the friction of the bogie 160 against the cam 120, but that no specific load bearing element is activated.

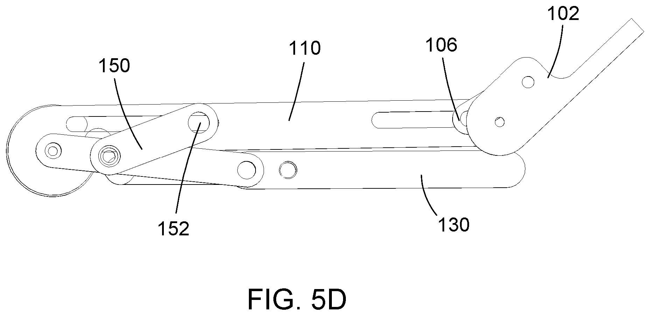

As shown in FIG. 5D, the body weight support 100 can be compactly collapsed, without compression of the spring, by rotating the lower member 130 to the second angular position relative to the upper member 110 with the toggle clip 102 in the open position. This means that the body weight support 100 can be easily folded, as the user does not have to overcome the resistance provided by the spring when folding the body weight support 100 with the toggle clip 102 in the open position.

The body weight support 100 described above provides the functionality to allow for location of the upper member 110 against the outer side of the user's upper leg while permitting connection of the lower member 130 to the rear of the user's boot, thereby reducing the loads on the user's muscles and joints without imparting an inward twisting force on the user's foot. This functionality is provided by the hinged lower member 130, particularly when the second portion 142 of the lower member 130 is in the first configuration. The body weight support 100 described above also provides the functionality to support the user's body weight when in a deep squat position (i.e. with a small angle between the upper member 110 and the lower member 130). This functionality is provided by the movement of the second portion 142 of the lower member 130 out of the first configuration when the lower member 130 is rotated sufficiently far towards the upper member 110. The ability to move the second portion 142 of the lower member 130 to the second configuration also means that the body weight support 100 can be compactly folded.

Variations or modifications to the systems and methods described herein are set out in the following paragraphs.

In the embodiment shown in FIGS. 1 to 5D, rotation of the bogie 160 is imparted by rotation of the lower member 130 towards the upper member 110. The rotation of the lower member 130 (specifically, the second portion 142 of the lower member 130) applies a tensile force to the bogie lever 170. This tensile force urges the bogie 160 to rotate, but the rotation of the bogie 160 is prevented until the lower member 130 is rotated to the angular position in which the first end 162 of the bogie 160 can move into the indent 122.

In an alternative embodiment, the first end 144 of the lower portion 142 can extend beyond the hinge 140, the first end 172 of the bogie lever 170 can be pivotally coupled to the first end 144 of the lower portion 142, and the second end 174 of the bogie lever 170 can be pivotally coupled to the first end 162 of the bogie 160. In such a configuration, the rotation of the second portion 142 applies a compressive force to the bogie lever 170. This compressive force on the first end 162 of the bogie 160 urges the bogie 160 to rotate, but the rotation of the bogie 160 is prevented until the lower member 130 is in the second angular position. In this position, the compressive force on the first end 162 of the bogie 160 causes the first end 162 of the bogie 160 to be pushed into the indent 122, thereby causing rotation of the bogie 160.

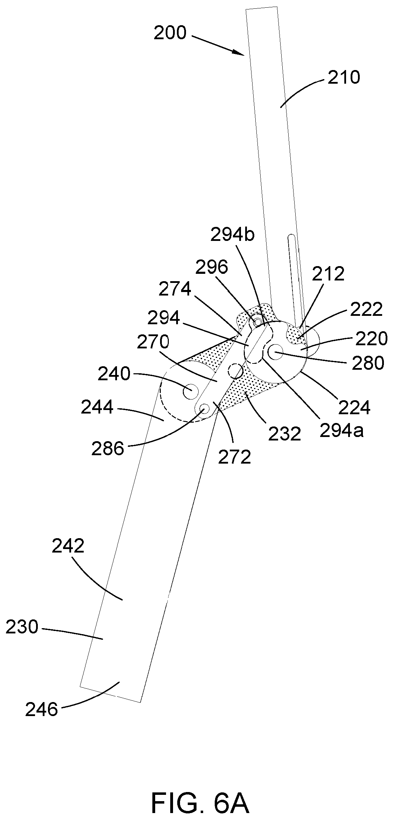

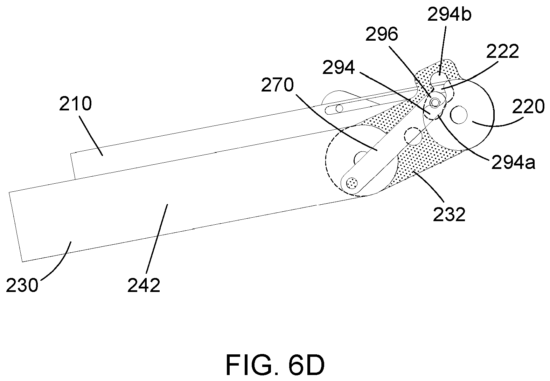

FIGS. 6A to 6D show an alternative pivotal connection of a body weight support 200 according to a second embodiment. As with the first embodiment, a first member in the form of an upper member 210 has a first end 212 that is fixedly coupled to a cam 220 having an indent 222 and a substantially constant radius surface 224.

A second member in the form of a lower member 230 comprises a first portion 232 and a second portion 242. The first portion 232 of the lower member 230 is pivotally coupled to the cam 220 about a first pivot 280 and pivotally coupled to the second portion 242 of the lower member 230 at a hinge 240.

The first portion 232 of the lower member 230 also includes a slot 294. The slot 294 comprises an annular portion 294a that has an outer radius that matches the radius of the substantially constant radius surface 224 of the cam 220, and is coincident with a portion of the cam surface. The slot 294 also includes a radial portion 294b that extends radially outwards from the end of the annular portion 294a that is closest to the indent 222. It will be appreciated that as the slot 294 is in the first portion 232 of the lower member 230, the slot 294, first pivot 280 and hinge 240 are in a fixed relationship with respect to one another.

The pivotal connection also includes a bearing 296 that is movable within the slot 294 such that the bearing 296 can move into the annular portion 294a and into the radial portion 294b of the slot 294 (depending on the angular position of the lower member 230 with respect to the upper member 210, as described below). The bearing 296 is configured to rotate as the lower member 230 is rotated relative to the upper member 210.

The body weight support 200 also includes a lever member 270 having a first end 272 that is pivotally coupled to the second portion 242 of the lower member 230. In particular, the first end 272 is pivotally coupled to the second portion 242 at a fourth pivot 286 located between the first end 244 and the second end 246 of the second portion 242. The second end 274 of the lever member is pivotally coupled to the bearing 296. When the second portion 242 of the lower member 230 is rotated, a tensile force is applied to the lever member 270. The tensile force via the lever member 270 pulls the bearing 296 against the slot 294.

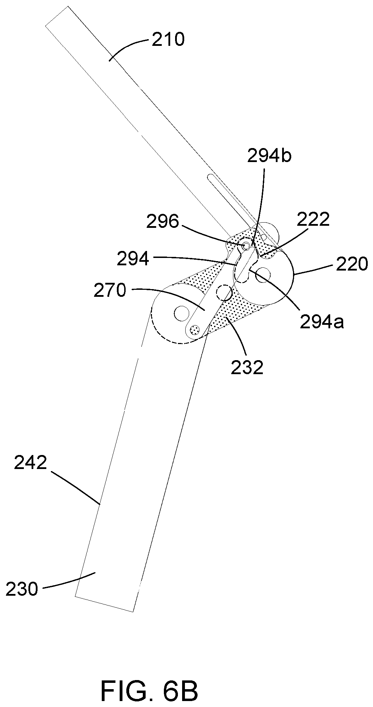

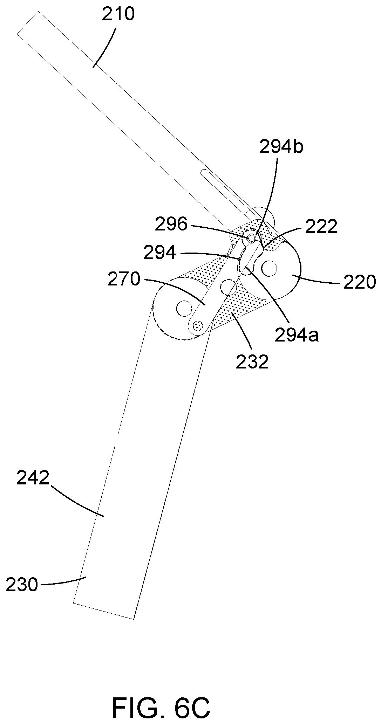

During rotation of the lower member 230 from the angular position shown in FIG. 6A to the angular position shown in FIG. 6B, the bearing 296 rotates within the radial portion 294b of the slot 294. Once the lower member 230 is rotated to the angular position shown in FIG. 6C, any further rotation of the lower member 230 towards the upper member 210 allows the bearing 296 to move radially inwards into the indent 222 in the surface of the cam 220. Once the bearing 296 has moved radially inwards into the indent 222, the bearing 296 can move within the annular portion 294a of the slot 294. Rotation of the lower member 230 towards the upper member 210 applies a tensile force to the lever member 270, which pulls the bearing 296 to the end of the annular portion 294a that is closest to the hinge 240 and causes the second portion 242 of the lower member 230 to move to its second configuration.

The pivotal coupling according to the second embodiment results in reduced friction force on the cam 220 compared to the friction exerted on the cam 120 of the body weight support 100 of the first embodiment. This is because some of the force applied via the lever member 270 is transferred to the slot 294 in the first portion 232 of the lower member 230, instead of the cam 220. The reduced friction on the cam 220 reduces the wear on the cam 220, which may prolong the life of the cam 220.

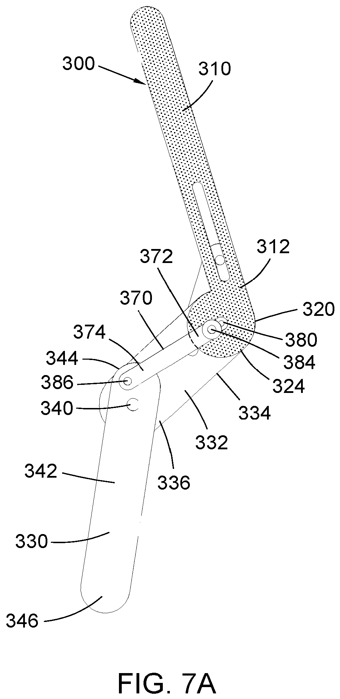

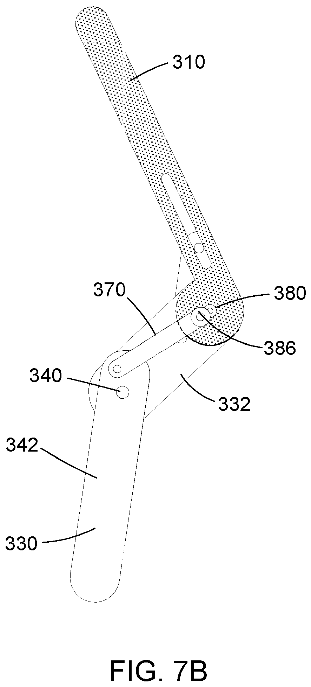

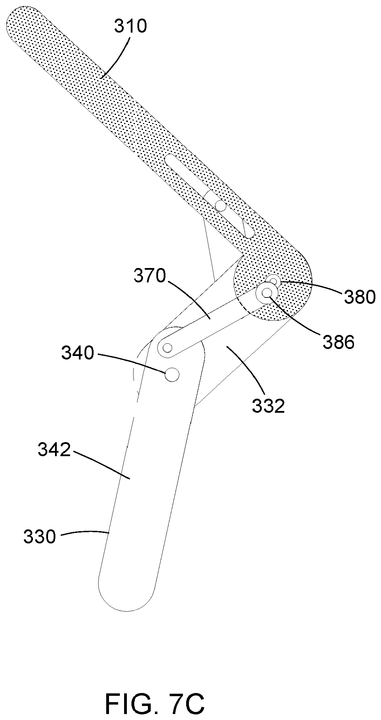

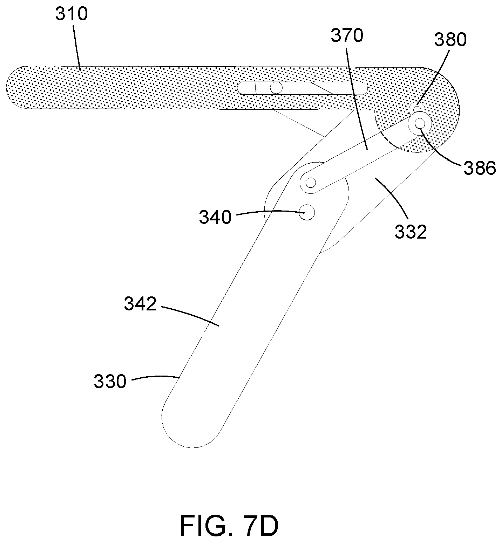

FIGS. 7A to 7E show a further alternative pivotal connection of a body weight support 300 according to a third embodiment. As with the first embodiment, a first member in the form of an upper member 310 has a first end 312 that is fixedly coupled to a cam 320. However, unlike the first and second embodiments, the cam 320 of the third embodiment has a substantially constant radius surface 324 and no indent.

A second member in the form of a lower member 330 comprises a first portion 332 and a second portion 342. The first portion 332 has a first end 334 pivotally coupled to the cam 320 at a first pivot 380, and a second end 336 coupled to a hinge 340. The second portion 342 has a first end 344 that extends beyond the hinge 340. This means that the hinge 340 is located towards the first end 344 of the second portion 342, but between the first end 344 and the second end 346.

The body weight support 300 also includes a lever member 370 having a first end 372 and a second end 374. The second end 374 of the lever member 370 is pivotally coupled to the cam 320 at a third pivot 384. The first end 372 of the lever member 370 is pivotally coupled to the first end 344 of the second portion 342 at a fourth pivot 386.

The third pivot 384 and the fourth pivot 386 are located such that the angle between the lever member 370 and the upper member 310 is smaller than the angle between the first portion 332 and the upper member 310. In addition, the third pivot 384 is radially offset from the first pivot 380 so that when the lower member 330 is at the first angular position, the third pivot 384 is closer to the hinge 340 than the first pivot 380.

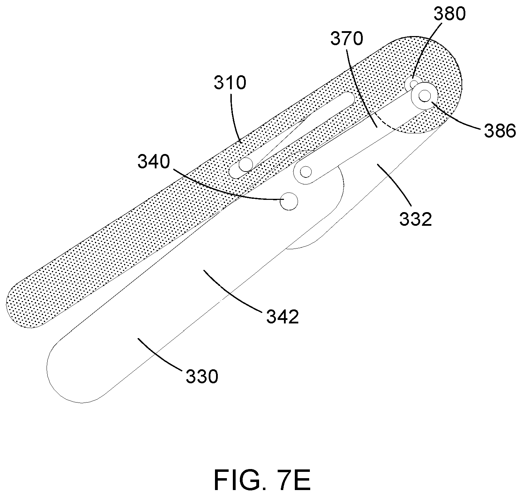

Unlike the body weight supports 100, 200 of the first and second embodiments, which only permit the second portion of the lower member to be moved from the first configuration to the second configuration when the lower member is at a particular angular position relative to the upper member, the second portion 342 of the lower member 330 of the body weight support 300 of the third embodiment moves constantly as the lower member 330 is rotated relative to the upper member 310. This is because the rotation of the lower member 330 relative to the upper member 310 causes rotation of the second portion 342 of the lower member 330 from the first configuration (FIG. 7A) to the second configuration (FIG. 7E).

Specifically, as shown in FIGS. 7A to 7E, the radial offset of the third pivot 384 from the first pivot 380 means that the third pivot 384 rotates about the first pivot 380 as the lower member 330 is rotated relative to the upper member 310. The rotation of the third pivot 384 about the first pivot 380 results in increased distance between the third pivot 384 and the hinge 340 as the lower member 330 is rotated. The increase in distance between the third pivot 384 and the hinge 340 allows the second portion 342 of the lower member 330 to be gradually straightened relative to the first portion 332 of the lower member 330 as the lower member 330 is rotated relative to the upper member 310.

The body weight supports 200, 300 of the second and third embodiments provide resistance to rotation of the lower member relative to the upper member, in the same way as for the body weight support 100 of the first embodiment. In addition, such resistance can be deactivated using a toggle clip in the same manner as for the body weight support 100 of the first embodiment.

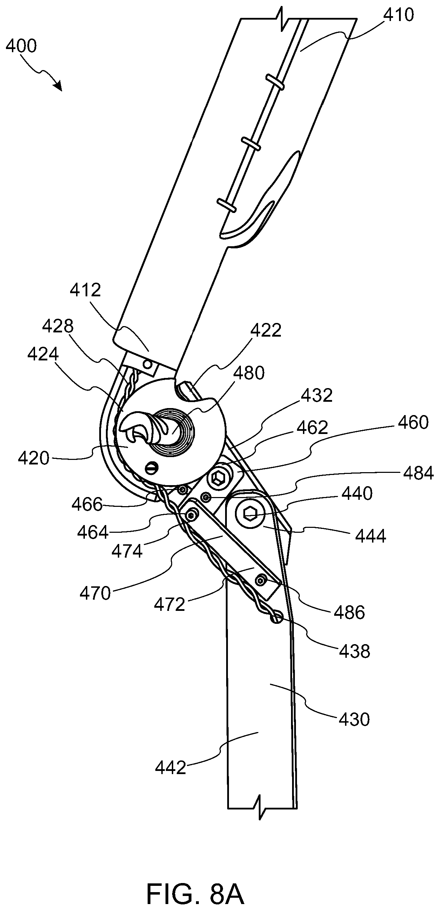

FIGS. 8A to 8J show a fourth embodiment of a body weight support 400, in the form of a modified version of the first embodiment described with reference to FIGS. 1 to 5D. As with the first embodiment, a first member in the form of an upper member 410 has a first end 412 that is fixedly coupled to a cam 420 having an indent 422 and a substantially constant radius surface 424.

A hinged second member in the form of a lower member 430 comprises a first portion 432 and a second portion 442. The first portion 432 of the lower member 430 is pivotally coupled to the cam 420 about a first pivot 480 and pivotally coupled to the second portion 442 of the lower member 430 at a hinge 440.

As with the first embodiment, the body weight support 400 also comprises a bogie 460 pivotally coupled to the first portion 432 of the lower member 430 at a second pivot 484 located between a first end 462 of the bogie 460 and a second end 464 of the bogie 460. However, unlike the first embodiment, the second end 464 of the bogie 460 of the fourth embodiment does not contact the substantially constant radius surface 424 of the cam 420. Instead, the second end 464 of the bogie 460 is in contact with a stop 466 that protrudes from the first portion 432 of the lower member 430. The stop 466 prevents the second end 464 of the bogie 460 from contacting the cam 420 when the bogie 460 is rotated about the second pivot 484. Consequently, the stop 466 reduces the friction force applied by the bogie 460 to the cam 420.

The body weight support 400 also includes a lever member in the form of a bogie lever 470, which extends between the second portion 442 of the lower member 430 and the second end 464 of the bogie 460. The bogie lever 470 has a first end 472 that is pivotally coupled to the second portion 442 of the lower member 430 at a third pivot 486 located between a first end 444 and a second end (not shown) of the second portion 442. A second end 474 of the bogie lever 470 is pivotally coupled to the second end 464 of the bogie 460.

In contrast to the first embodiment, the body weight support 400 of the fourth embodiment does not include a connection member that is pivotally coupled to the first portion 432 of the lower member 430. Instead, the body weight support 400 includes a load bearing element comprising a replaceable cable 428. A first end 438 of the cable 428 is attached to the second portion 442 of the lower member 430 adjacent to the third pivot 486. Specifically, the first end 438 of the cable 428 is attached to the second portion 442 at a position such that the distance between the first end 438 of the cable 428 and the hinge 440 is greater than the distance between the third pivot 486 and the hinge 440. In other words, the first end 438 of the cable 428 is attached to the second portion 442 at a point that is more distal than the third pivot 486. Preferably, the tangential distance between the hinge 440 and the cable 428 is slightly greater than the radius of a pulley (described further below) that is coincident with the cam 420. This ensures full rotation of the lower member 430 (specifically, the first portion 432 of the lower member 430) relative to the upper member 410 prior to rotation of the second portion 442 of the lower member 430 about the hinge 440.

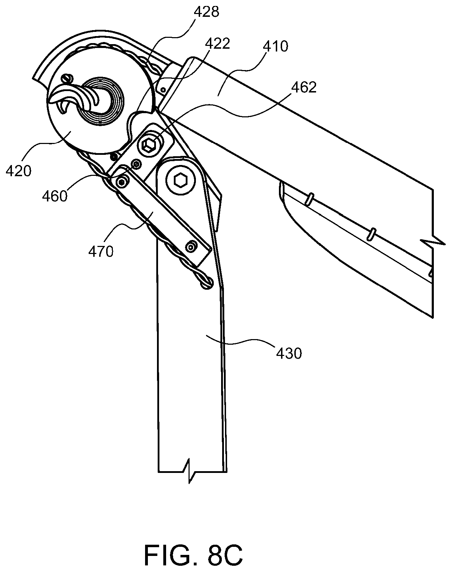

The load bearing element also includes a spring (not shown), which is housed within the upper member 410. A second end (not shown) of the cable 428 is attached to the end of the spring that is furthest from the cam 420, meaning that the cable 428 protrudes from an opening in the first end 412 of the upper member 410. The spring is compressed when the cable 428 is pulled out of the first end 412 (i.e. as the lower member 430 is rotated relative to the upper member 410 from the angular position shown in FIG. 8A to the angular position shown in FIG. 8C, for example).

The cable 428 is in contact with a pulley (not shown) that is coincident with the cam 420. While the cam 420 is fixedly connected to the upper member 410, the pulley is free to rotate under the action of the cable 428 as the lower member 430 is rotated relative to the upper member 410, such that the cable 428 does not slide over the pulley. Although not shown in FIG. 8A, a sandwich construction may be used, in which the pulley is disposed between two symmetrical cams 420. The pulley is offset from the cam 420 in the axial direction (i.e. into the drawing shown in FIG. 8A), so that it does not interfere with the rotation of the first end 462 of the bogie 460 into the indent 422. The pulley radius may be substantially equal to the radius of the substantially constant radius surface 424 of the cam 420.

As described above, the bogie lever 170 of the body weight support 100 of the first embodiment is under tension when the second portion 142 of the lower member 130 is rotated towards the upper member 110. The tension applied to the bogie lever 170 urges the first end 162 of the bogie 160 to rotate towards the first pivot 180, which results in a frictional force being applied to the cam 120 by the first end 162 of the bogie 160. In contrast, the tensile force applied by the cable 428 of the body weight support 400 of the fourth embodiment means that the tension applied to the bogie lever 470 is reduced or even eliminated. Consequently, the first end 462 of the bogie 460 is not urged to rotate towards the first pivot 480, meaning that the frictional force applied to the cam 420 by the first end 462 of the bogie 460 is reduced or even eliminated. This reduces the frictional force on the cam 420, thereby reducing wear on the cam 420.

In some examples, the force applied by the cable 428 may be sufficiently high that a compressive force is applied to the bogie lever 470, thereby urging the first end 462 of the bogie 460 to rotate away from the first pivot 480. However, as explained above, rotation of the bogie 460 in this manner is prevented by the abutment of the second end 464 of the bogie 460 against the stop 466.

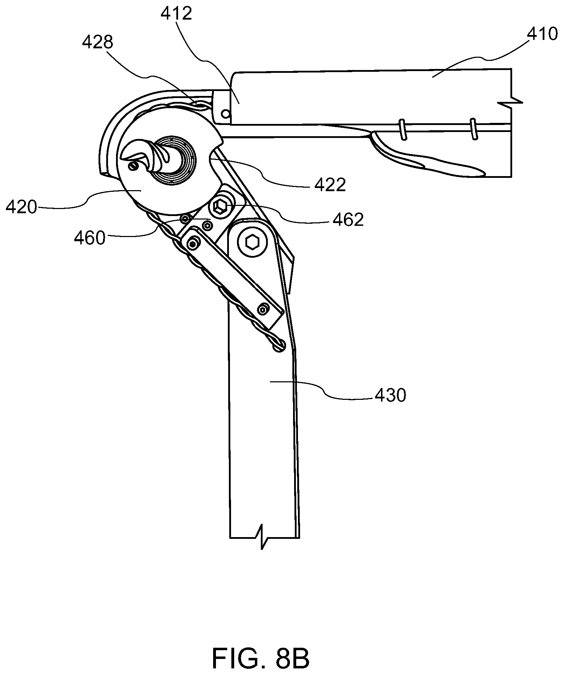

FIG. 8A shows the lower member 430 in a first angular position relative to the upper member 410. Rotation of the lower member 430 anticlockwise relative to the upper member 410 (in the orientation shown in FIG. 8A) causes movement of the lower member 430 to the position shown in FIG. 8B, in which the first end 462 of the bogie 460 is adjacent to the indent 422 in the cam 420.

When moving from the position shown in FIG. 8A to the position shown in FIG. 8B, the contact between the cable 428 and the pulley means that the cable 428 is pulled out of the first end 412 of the upper member 410 as the lower member 430 is rotated towards the upper member 410. Therefore, the length of cable 428 protruding from the first end 412 of the upper member 410 in the position shown in FIG. 8B exceeds the length of cable 428 protruding from the first end 412 in the position shown in FIG. 8A, meaning that the spring within the upper member 410 is compressed as the lower member 430 is moved towards the upper member 410. The compression of the spring resists the rotational movement of the lower member 430 from the angular position relative to the upper member 410 shown in FIG. 8A to the angular position relative to the upper member 410 shown in FIG. 8B. In other words, the compression of the spring caused by drawing the cable 428 out of the first end 412 of the upper member 410 resists the rotational movement of the lower member 430 towards the upper member 410.

As shown in FIG. 8C, further rotation of the lower member 430 relative to the upper member 410 does not result in the first end 462 of the bogie 460 being rotated into the indent 422 in the cam 420 (unlike the body weight support 100 of the first embodiment). This is because the tensile force applied by the cable 428 eliminates the tension in the bogie lever 470, meaning that the first end 462 of the bogie 460 is not being urged to rotate into the indent 422.

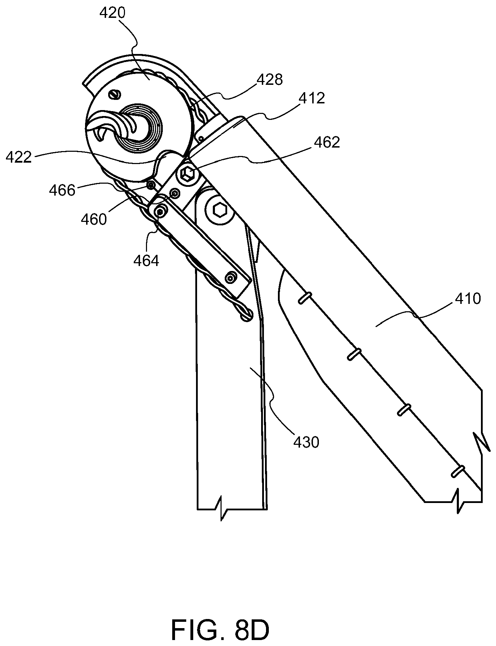

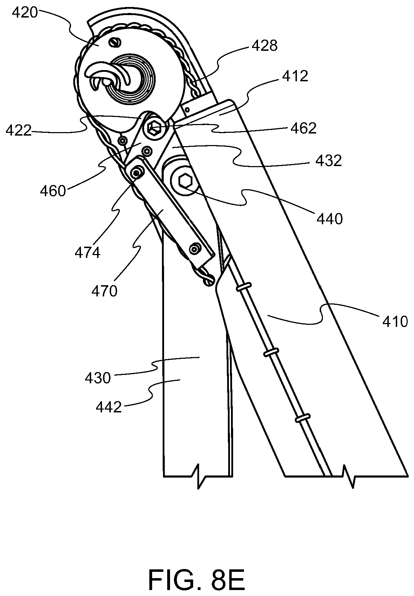

Instead, the lower member 430 is rotated to the position shown in FIG. 8D, in which the first end 462 of the bogie 460 contacts the first end 412 of the upper member 410. The first end 412 of the upper member 410 is fixedly connected to the cam 420 at an angular position that is immediately adjacent to the indent 422, meaning that the contact between the first end 412 of the upper member 410 and the first end 462 of the bogie 460 forces the first end 462 of the bogie 460 into the indent 422 (as shown in FIG. 8E). Rotation of the first end 462 of the bogie 460 in the opposite direction (i.e. away from the indent 422) is prevented by the stop 466, which abuts the second end 464 of the bogie 460. As mentioned above, this means that no force from the second end 464 of the bogie 460 is applied to the cam 420.

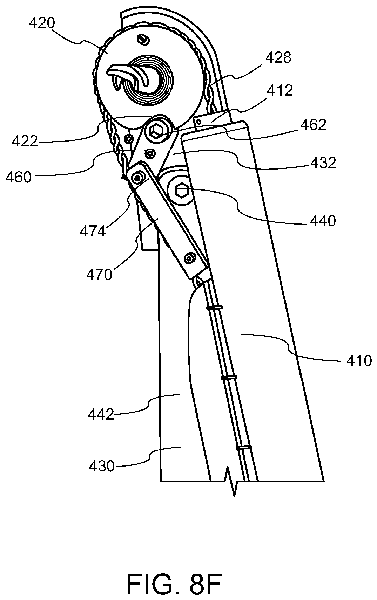

In the position shown in FIG. 8E, the first end 462 of the bogie 460 has been forced into the indent 422 by the action of the first end 412 of the upper member 410. This means that the second end 474 of the bogie lever 470 is rotated towards the hinge 440, thereby allowing the second portion 442 of the lower member 430 to be rotated relative to the first portion 432 of the lower member 430 (i.e. moved from a first configuration shown in FIGS. 8A to 8D to a second configuration shown in FIG. 8F). The rotation of the second portion 442 of the lower member 430 relative to the first portion 432 of the lower member 430 is as described above for the body weight support 100 of the first embodiment. As will be appreciated, rotation of the lower member 430 from the angular position relative to the upper member 410 shown in FIG. 8B to the angular position relative to the upper member 410 shown in FIG. 8E draws additional cable 428 out of the first end 412 of the upper member 410, further compressing the spring, resulting in further resistance to such rotation.

As shown in FIGS. 8D to 8F, the tangential distance between the cable 428 and the hinge 440 reduces as the second portion 442 of lower member 430 is rotated relative to the first portion 432 of the lower member 430 from the angular position shown in FIG. 8D to the angular position shown in FIG. 8E. The tangential distance between the cable 428 and the hinge 440 is further reduced when the second portion 442 is moved from the angular position relative to the first position 432 shown in FIG. 8E to the relative angular position shown in FIG. 8F.

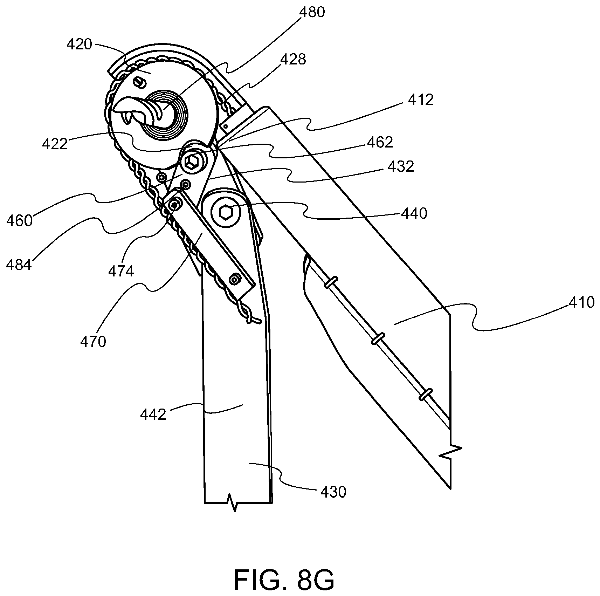

The reduction in the tangential distance between the cable 428 and the hinge 440 means that when the lower member 430 is rotated in the opposite direction (i.e. away from the upper member 410, by rotation from the angular position shown in FIG. 8F to the angular position shown in FIG. 8G), the first portion 432 of the lower member 430 pivots relative to the upper member 410 about the first pivot 480 before the second portion 442 of the lower member 430 hinges relative to the first portion 432 of the lower member 430 about the hinge 440. This is because the moment about the first pivot 480 exceeds the moment about the hinge 440 (as a result of the radius of the pulley exceeding the tangential distance between the cable 428 and the hinge 440).

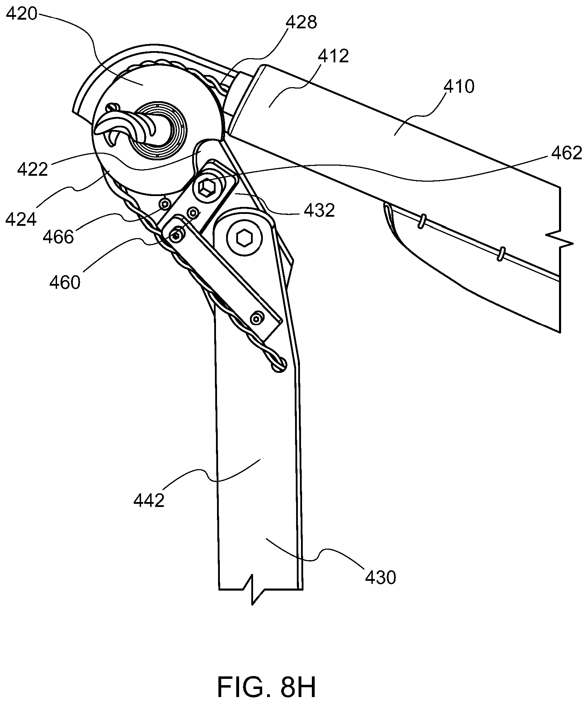

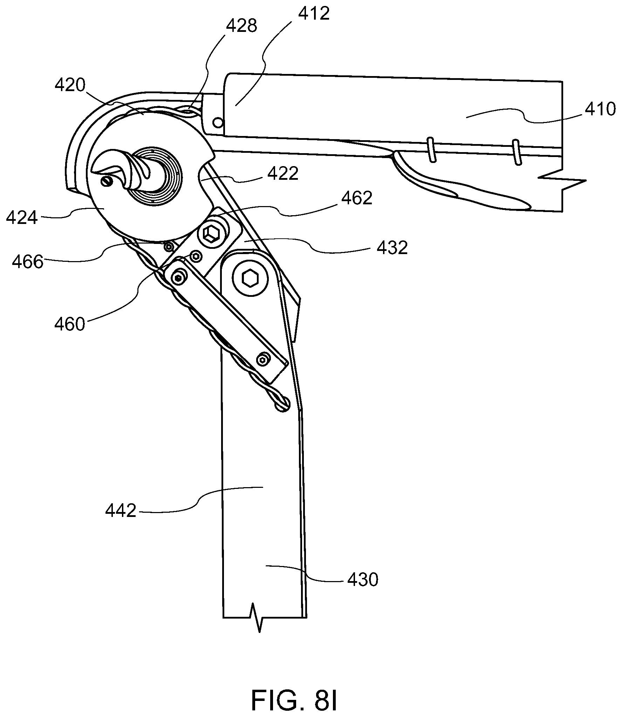

When the lower member 430 is moved away from the upper member 410 by angular rotation in the opposite direction (i.e. from the angular position shown in FIG. 8F to the angular position shown in FIG. 8G), the indent 422 forces the first end 462 of the bogie 460 to rotate away from the first pivot 480. The surface of the indent 422 located furthest from the attachment point of the first end 412 of the upper member 410 may be curved in order to provide a smooth transition of the first end 462 of the bogie 460 out of the indent 422, as shown in FIGS. 8A to 8J. The rotation of the first end 462 of the bogie 460 out of the indent 422 causes the bogie 460 to rotate about the second pivot 484, which forces the second end 474 of the bogie lever 470 to rotate away from the hinge 440. The rotation of the bogie lever 470 away from the hinge 440 drives rotation of the second portion 442 of the lower member 430 relative to the first portion 432 of the lower member 430 (i.e. from the second configuration shown in FIGS. 8F to the first configuration shown in FIGS. 8H and 8I).

The displacement of the first end 462 of the bogie 460 out of the indent 422 means that the second portion 442 of the lower member 430 is moved from the second configuration to the first configuration prior to significant rotation of the upper member 410 relative to the first portion 432 of the lower member 430 (as shown in FIG. 8H). Therefore, the portions 432, 442 of the lower member 430 do not straighten until the first portion 432 has been fully rotated towards the upper member 410, and the portions 432, 442 are returned to the unstraightened configuration prior to significant rotation of the upper member 410 relative to the first portion 432.

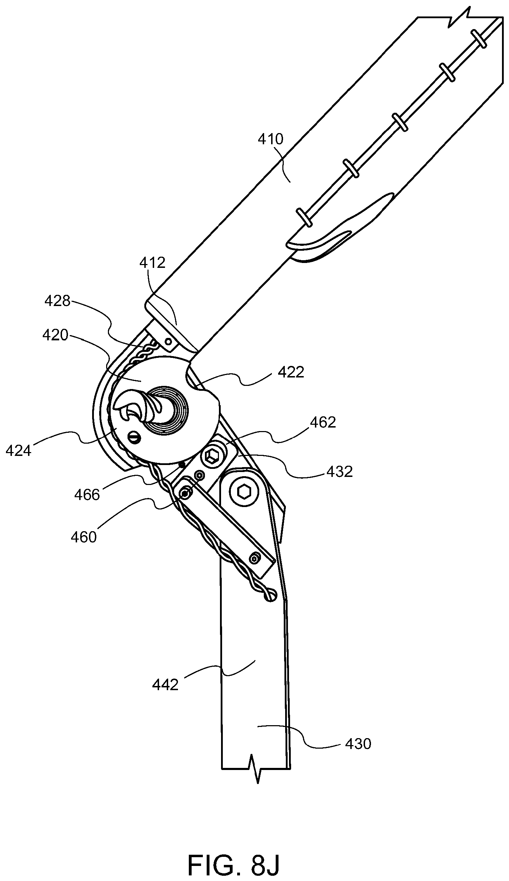

Further rotation of the lower member 430 from the angular position relative to the upper member 410 shown in FIG. 8H to the angular position relative to the upper member 410 shown in FIG. 8J results in the cable 428 being pulled back through the first end 412 of the upper member 410, reducing compression on the spring, and thereby reducing resistance to angular rotation of the lower member 430 towards the upper member 410. The second portion 442 of the lower member 430 stays in the second configuration during rotation of the lower member 430 away from the upper member 410 because the rotation of the bogie 460 is restricted by the substantially constant radius surface 424 of the cam 420 and the stop 466.

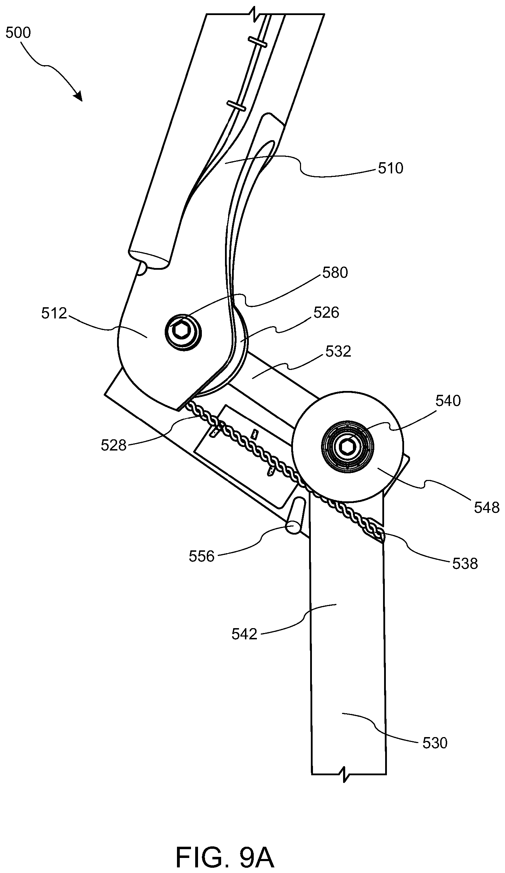

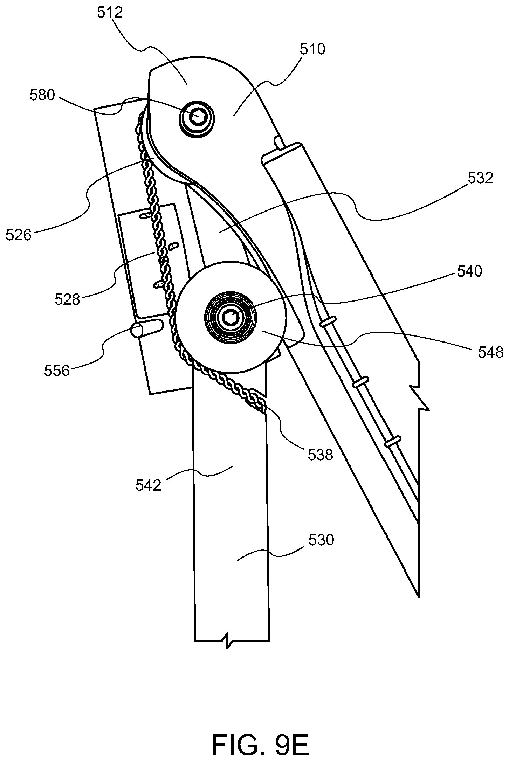

FIGS. 9A to 9F show a fifth embodiment of a body weight support 500. The body weight support 500 includes an upper member 510 having a first end 512 housing a first pulley 526. The body weight support 500 also includes a lower member 530 comprising a first portion 532 and a second portion 542. The first portion 532 of the lower member 530 is pivotally coupled to the first end 512 of the upper member 510 about a first pivot 580 and pivotally coupled to the second portion 542 of the lower member 530 about a hinge 540. A second pulley 548 is coincident with the hinge 540.

The first portion 532 of the lower member 530 also includes a stop 556 that protrudes outwardly from the first portion 532 so that it is capable of abutting the second portion 542 when the second portion 542 is rotated into contact with it (i.e. as shown in FIG. 9A). The stop 556 limits the extent of rotation of the second portion 542 away from the upper member 510 about the hinge 540.

As with the fourth embodiment, the body weight support 500 includes a load bearing element comprising a replaceable cable 528. A first end 538 of the cable 528 is attached to the second portion 542 of the lower member 530 such that when the second portion 542 abuts the stop 556, the tangential distance between the hinge 540 and the cable 528 exceeds the radius of the first pulley 526. This means that, when the body weight support 500 is in the configuration shown in FIG. 9A, the cable 528 is not in contact with the second pulley 548 (i.e. there is a gap between the cable 528 and the second pulley 548).

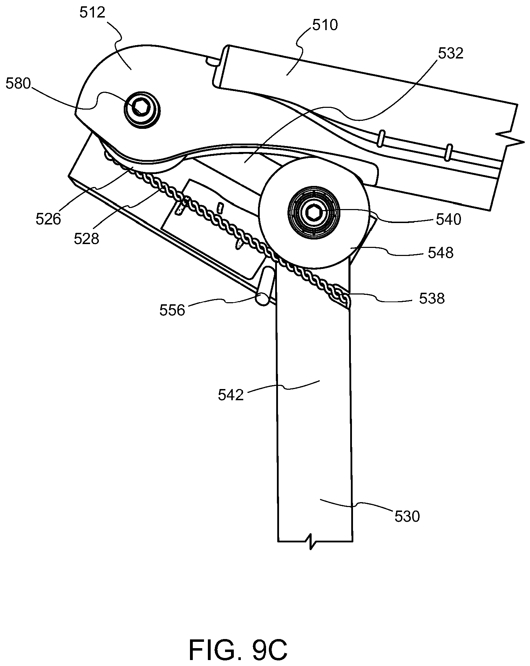

The load bearing element also includes a spring (not shown), which is housed within the upper member 510. A second end (not shown) of the cable 528 is attached to the end of the spring that is furthest from the first pulley 526, meaning that the cable 528 protrudes from an opening in the first end 512 of the upper member 510. The spring is compressed when the cable 528 is pulled out of the first end 512 (i.e. as the lower member 530 is rotated relative to the upper member 510 from the angular position shown in FIG. 9A to the angular position shown in FIG. 9C, for example).

The force applied by the cable 528 to the second portion 542 of the lower member 530 pulls the second portion 542 of the lower member 530 away from the upper member 510. As mentioned above, the stop 556 limits the extent of rotation of the second portion 542 away from the upper member 510.

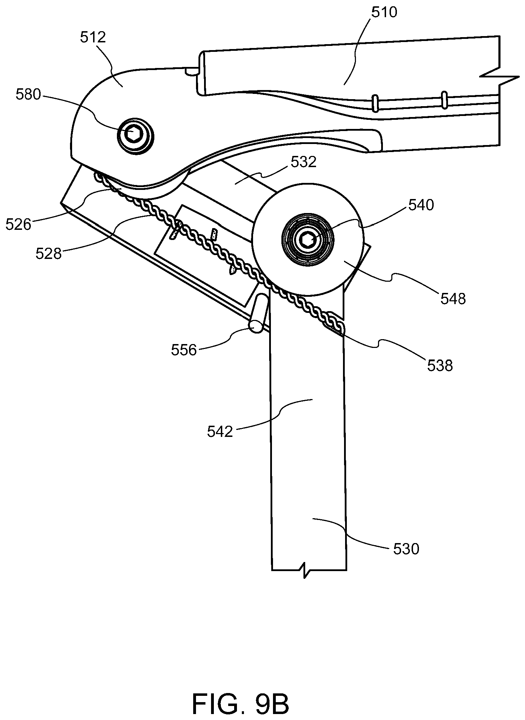

When the body weight support 500 is in the configuration shown in FIG. 9A, the cable 528 is in contact with the first pulley 526 and is not in contact with the second pulley 548. This means that when the lower member 530 (specifically, the second portion 542 of the lower member 530) is rotated relative to the upper member 510 to the angular position shown in FIG. 9B, the lower member 530 pivots about the first pivot 580 and not the hinge 540.

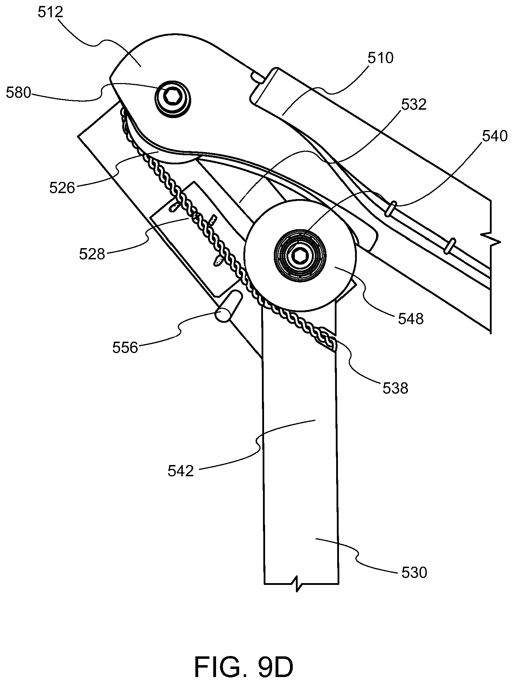

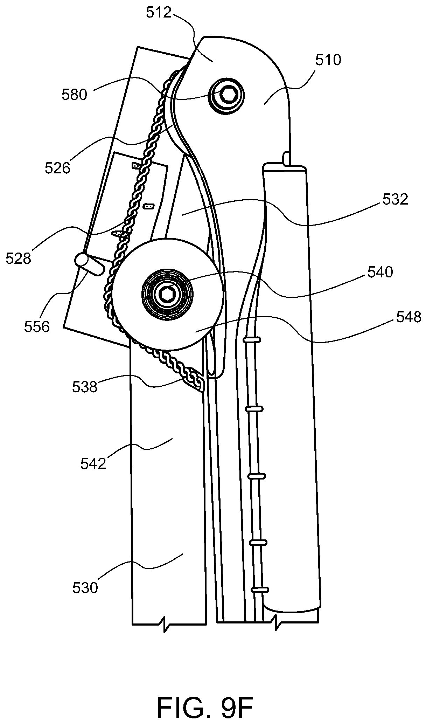

Further rotation of the lower member 530 towards the upper member 510 brings the first portion 532 of the lower member 530 into contact with the upper member 510 (as shown in FIG. 9C). At this point, the first portion 532 of the lower member 530 cannot rotate further towards the upper member 510, meaning that further rotation of the second portion 542 of the lower member 530 towards the upper member 510 causes rotation of the second portion 542 relative to the first portion 532 about the hinge 540. This rotation moves the second portion 542 out of contact with the stop 556 and brings the cable 528 into contact with the second pulley 548 (FIG. 9D). Further rotation of the second portion 542 relative to the first portion 532 about the hinge 540 results in movement of the second portion 542 from the first configuration relative to the first portion 532 shown in FIGS. 9A to 9C to the second configuration relative to the first portion 532 shown in FIG. 9F.

In the first, second and fourth embodiments of the body weight support, a user is unable to rotate the second portion of the lower member relative to the first portion of the lower member until the lower member is in a particular angular position relative to the upper member. In contrast, a user of the body weight support 500 of the fifth embodiment is able to move the second portion 542 of the lower member 530 relative to the first portion 532 of the lower member 530 before the lower member 530 is in a particular angular position relative to the upper member 510. This could be done, for example, by the user holding the first portion 532 and the second portion 542 and rotating them relative to one another. However, when the body weight support 500 of the fifth embodiment is in use, the rotation of the second portion 542 of the lower member 530 relative to the upper member 510 does not cause the second portion 542 of the lower member 530 to rotate relative to the first portion 532 of the lower member 530 until the lower member 530 (specifically the first portion 532 of the lower member 530) is in a particular position (shown in FIG. 9C) relative to the upper member 510. The same is also true of the body weight supports of the first, second and fourth embodiments.

In the body weight supports 400, 500 of the fourth and fifth embodiments, the load bearing element may be deactivated by decoupling the second end of the cable 428, 528 from the spring housed within the upper member 410, 510. This allows the cable 428, 528 to move freely relative to the spring, meaning that the spring does not resist relative movement of the lower member 430, 530 and the upper member 410, 510. The load bearing element can be reactivated by recoupling the second end of the cable 428, 528 to the spring.

The singular terms "a" and "an" should not be taken to mean "one and only one". Rather, they should be taken to mean "at least one" or "one or more" unless stated otherwise. The word "comprising" and its derivatives including "comprises" and "comprise" include each of the stated features, but does not exclude the inclusion of one or more further features. The terms "upper" and "lower" should not be taken to limit the positions of the features that they describe. Instead, such terms are used for the reader's convenience and by way of example only. Skilled persons will appreciate that the devices described herein may be used in a number of different orientations in which the relative positions of the features differ from the positions described herein using the terms "upper" and "lower".

The above implementations have been described by way of example only, and the described implementations are to be considered in all respects only as illustrative and not restrictive. It will be appreciated that variations of the described implementations may be made without departing from the scope of the invention which is solely defined by the appended claims.

## Claims

1. A body weight support (100), comprising:
a first member (110) configured for location against a first portion of a limb of a user, the first portion of the limb being on a first side of a joint of the user;
a second member (130) configured for location against a second portion of the limb on a second side of the joint of the user, wherein the second member is rotatable relative to the first member about a pivot (180); and
a load bearing element configured to provide a resistance to rotation of the second member relative to the first member;
wherein the second member comprises:
a first portion (132) proximate to the pivot (180); and
a second portion (142) extending from the first portion (132), distal to the pivot (180);
wherein the second portion (142) is movable from a first configuration in which a first angle between the first portion (132) and the first member is less than a second angle between the second portion (142) and the first member, to a second configuration in which the difference between the first angle and the second angle is less than the difference between the first angle and the second angle in the first configuration;
wherein the second member is rotatable from a first angular position relative to the first member to a second angular position relative to the first member, wherein during rotation of the second member from the first angular position to the second angular position, the second portion (142) is prevented from moving relative to the first portion (132) until the second member is in the second angular position.

2. A body weight support according to claim 1, wherein rotation of the second portion (142) of the second member relative to the first member does not cause movement of the second portion (142) from the first configuration to the second configuration when the second member is in the first angular position, and wherein rotation of the second portion (142) of the second member relative to the first member causes movement of the second portion (142) from the first configuration to the second configuration when the second member is in the second angular position.

3. A body weight support according to claim 1 or claim 2, wherein when the second member is in the second angular position, an angle between the first portion (132) and the first member is smaller than when the second member is in the first angular position, wherein when the second member is in the second angular position, the first portion (132) is optionally substantially parallel to the first member.

4. A body weight support according to any of claims 1 to 3, wherein when the second portion (142) is in the second configuration, the second portion (142) is in line with the first portion (132).

5. A body weight support according to any of claims 1 to 4, wherein the second portion (142) is hingedly coupled to the first portion (132).

6. A body weight support according to any of claims 1 to 5, wherein the length of the second portion (142) is adjustable.

7. A body weight support according to any of claims 1 to 6, wherein the length of the first portion (132) is adjustable.

8. A body weight support according to any of claims 1 to 7, wherein an angle between the first portion (132) and the second portion (142) in the first configuration of the second member is adjustable.

9. A body weight support according to any of claims 1 to 8, further comprising a pivotal coupling configured to permit the rotation of the second member relative to the first member, wherein the body weight support optionally further comprises:
a lever member coupled to the second member, wherein the lever member (270) is configured to apply a frictional force to the pivotal coupling during rotation of the second member from the first angular position to the second angular position.

10. A body weight support according to any of claims 1 to 9, wherein the load bearing element is deactivatable to permit substantially free rotation of the second member relative to the first member.

11. A body weight support according to any of claims 1 to 10, wherein the first member is configured for location against an upper leg of the user and the second member is configured for location against a lower leg of the user, wherein the first member is optionally configured for location against the outer side of the user's upper leg.

12. A body weight support according to claim 11, wherein the second portion (142) is connectable to a boot of the user, wherein the second portion (142) is optionally connectable to the rear of the boot of the user.

13. A body weight support according to any of claims 1 to 12, further comprising a connection member (150) pivotally coupled to the first portion (132) of the second member, wherein the connection member (150) engages an end of the load bearing element, wherein the length of the connection member (150) is optionally adjustable.

14. A body weight support according to claim 13, wherein an end of the connection member (150) is slidably movable within a slot (116) in the first member, and wherein the end of the connection member (150) engages the end of the load bearing element.

15. A body weight support according to any of claims 1 to 12, wherein the load bearing element comprises a cable (428, 528), wherein a first end (438) of the cable (428, 528) is optionally attached to the second portion (142) of the second member.

## Patentansprüche

1. Körpergewichtsstütze (100), umfassend:
ein erstes Glied (110), das zum Anordnen an einem ersten Abschnitt einer Gliedmaße eines Benutzers ausgestaltet ist, wobei sich der erste Abschnitt der Gliedmaße an einer ersten Seite eines Gelenks des Benutzers befindet, ein zweites Glied (130), das zum Anordnen an einem zweiten Abschnitt der Gliedmaße an einer zweiten Seite des Gelenks des Benutzers ausgestaltet ist, wobei das zweite Glied um einen Drehpunkt (180) relativ zu dem ersten Glied drehbar ist, und
ein Tragelement, das zum Bieten von Widerstand gegen eine Drehung des zweiten Glieds relativ zu dem ersten Glied ausgestaltet ist,
wobei das zweite Glied Folgendes umfasst:
einen ersten Abschnitt (132) nahe dem Drehpunkt (180) und einen zweiten Abschnitt (142), der sich von dem ersten Abschnitt (132) distal zu dem Drehpunkt (180) erstreckt, wobei der zweite Abschnitt (142) aus einer ersten Konfiguration, in der ein erster Winkel zwischen dem ersten Abschnitt (132) und dem ersten Glied kleiner als ein zweiter Winkel zwischen dem zweiten Abschnitt (142) und dem ersten Glied ist, in eine zweite Konfiguration bewegbar ist, in der die Differenz zwischen dem ersten Winkel und dem zweiten Winkel kleiner als die Differenz zwischen dem ersten Winkel und dem zweiten Winkel in der ersten Konfiguration ist,
wobei das zweite Glied aus einer ersten Winkelposition relativ zu dem ersten Glied in eine zweite Winkelposition relativ zu dem ersten Glied drehbar ist, wobei während der Drehung des zweiten Glieds aus der ersten Winkelposition in die zweite Winkelposition dafür gesorgt wird, dass sich der zweite Abschnitt (142) erst dann relativ zu dem ersten Abschnitt (132) bewegt, wenn das zweite Glied in der zweiten Winkelposition ist.

2. Körpergewichtsstütze nach Anspruch 1, wobei eine Drehung des zweiten Abschnitts (142) des zweiten Glieds relativ zu dem ersten Glied keine Bewegung des zweiten Abschnitts (142) aus der ersten Konfiguration in die zweite Konfiguration veranlasst, wenn das zweite Glied in der ersten Winkelposition ist, und wobei eine Drehung des zweiten Abschnitts (142) des zweiten Glieds relativ zu dem ersten Glied eine Bewegung des zweiten Abschnitts (142) aus der ersten Konfiguration in die zweite Konfiguration veranlasst, wenn das zweite Glied in der zweiten Winkelposition ist.

3. Körpergewichtsstütze nach Anspruch 1 oder Anspruch 2, wobei, wenn das zweite Glied in der zweiten Winkelposition ist, ein Winkel zwischen dem ersten Abschnitt (132) und dem ersten Glied kleiner ist als wenn das zweite Glied in der ersten Winkelposition ist, wobei der erste Abschnitt (132) optional im Wesentlichen parallel zu dem ersten Glied verläuft, wenn das zweite Glied in der zweiten Winkelposition ist.

4. Körpergewichtsstütze nach einem der Ansprüche 1 bis 3, wobei der zweite Abschnitt (142) mit dem ersten Abschnitt (132) fluchtet, wenn der zweite Abschnitt (142) in der zweiten Konfiguration ist.

5. Körpergewichtsstütze nach einem der Ansprüche 1 bis 4, wobei der zweite Abschnitt (142) gelenkig an den ersten Abschnitt (132) gekoppelt ist.

6. Körpergewichtsstütze nach einem der Ansprüche 1 bis 5, wobei die Länge des zweiten Abschnitts (142) verstellbar ist.

7. Körpergewichtsstütze nach einem der Ansprüche 1 bis 6, wobei die Länge des ersten Abschnitts (132) verstellbar ist.

8. Körpergewichtsstütze nach einem der Ansprüche 1 bis 7, wobei ein Winkel zwischen dem ersten Abschnitt (132) und dem zweiten Abschnitt (142) in der ersten Konfiguration des zweiten Glieds verstellbar ist.

9. Körpergewichtsstütze nach einem der Ansprüche 1 bis 8, ferner umfassend eine Schwenkkopplung, die dazu ausgestaltet ist, die Drehung des zweiten Glieds relativ zu dem ersten Glied zu gestatten, wobei die Körpergewichtsstütze optional ferner Folgendes umfasst: ein an das zweite Glied gekoppeltes Hebelglied, wobei das Hebelglied (270) dazu ausgestaltet ist, während der Drehung des zweiten Glieds aus der ersten Winkelposition in die zweite Winkelposition eine Reibkraft auf die Schwenkkopplung auszuüben.

10. Körpergewichtsstütze nach einem der Ansprüche 1 bis 9, wobei das Tragelement deaktiviert werden kann, um eine im Wesentlichen freie Drehung des zweiten Glieds relativ zu dem ersten Glied zu gestatten.

11. Körpergewichtsstütze nach einem der Ansprüche 1 bis 10, wobei das erste Glied zum Anordnen an einem Oberschenkel des Benutzers ausgestaltet ist und das zweite Glied zum Anordnen an einem Unterschenkel des Benutzers ausgestaltet ist, wobei das erste Glied optional zum Anordnen an der Außenseite des Oberschenkels des Benutzers ausgestaltet ist.

12. Körpergewichtsstütze nach Anspruch 11, wobei der zweite Abschnitt (142) mit einem Stiefel des Benutzers verbindbar ist, wobei der zweite Abschnitt (142) optional mit der Rückseite des Stiefels des Benutzers verbindbar ist.

13. Körpergewichtsstütze nach einem der Ansprüche 1 bis 12, ferner umfassend ein Verbindungsglied (150), das schwenkbar an den ersten Abschnitt (132) des zweiten Glieds gekoppelt ist, wobei das Verbindungsglied (150) ein Ende des Tragelements in Eingriff nimmt, wobei die Länge des Verbindungsglieds (150) optional verstellbar ist.

14. Körpergewichtsstütze nach Anspruch 13, wobei ein Ende des Verbindungsglieds (150) verschiebbar in einem Schlitz (116) in dem ersten Glied beweglich ist und wobei das Ende des Verbindungsglieds (150) das Ende des Tragelements in Eingriff nimmt.

15. Körpergewichtsstütze nach einem der Ansprüche 1 bis 12, wobei das Tragelement ein Seil (428, 528) umfasst, wobei ein erstes Ende (438) des Seils (428, 528) optional an dem zweiten Abschnitt (142) des zweiten Glieds angebracht ist.

## Revendications

1. Support de poids corporel (100), comprenant :
un premier élément (110) configuré pour être placé contre une première partie d'un membre d'un utilisateur, la première partie du membre étant sur un premier côté d'une articulation de l'utilisateur ;
un second élément (130) configuré pour être placé contre une seconde partie du membre sur un second côté de l'articulation de l'utilisateur, le second élément pouvant tourner par rapport au premier élément autour d'un pivot (180) ; et
un élément porteur configuré pour offrir une résistance à la rotation du second élément par rapport au premier élément ;
dans lequel le second élément comprend :
une première partie (132) proche du pivot (180) ; et
une seconde partie (142) s'étendant à partir de la première partie (132), éloignée du pivot (180) ;
dans lequel la seconde partie (142) est mobile d'une première configuration, dans laquelle un premier angle entre la première partie (132) et le premier élément est inférieur à un second angle entre la seconde partie (142) et le premier élément, à une seconde configuration, dans laquelle la différence entre le premier angle et le second angle est inférieure à la différence entre le premier angle et le second angle dans la première configuration ;
dans lequel le second élément peut tourner d'une première position angulaire par rapport au premier élément à une seconde position angulaire par rapport au premier élément, un déplacement de la seconde partie (142) par rapport à la première partie (132) étant bloqué, pendant la rotation du second élément de la première position angulaire à la seconde position angulaire, jusqu'à ce que le second élément se trouve dans la seconde position angulaire.

2. Support de poids corporel selon la revendication 1, dans lequel la rotation de la seconde partie (142) du second élément par rapport au premier élément ne provoque pas le déplacement de la seconde partie (142) de la première configuration à la seconde configuration lorsque le second élément est dans la première position angulaire, et dans lequel la rotation de la seconde partie (142) du second élément par rapport au premier élément provoque le déplacement de la seconde partie (142) de la première configuration à la seconde configuration lorsque le second élément est dans la seconde position angulaire.

3. Support de poids corporel selon la revendication 1 ou la revendication 2, dans lequel, lorsque le second élément est dans la seconde position angulaire, un angle entre la première partie (132) et le premier élément est plus petit que lorsque le second élément est dans la première position angulaire, dans lequel, lorsque le second élément est dans la seconde position angulaire, la première partie (132) est facultativement sensiblement parallèle au premier élément.

4. Support de poids corporel selon l'une quelconque des revendications 1 à 3, dans lequel, lorsque la seconde partie (142) se trouve dans la seconde configuration, la seconde partie (142) est alignée avec la première partie (132).

5. Support de poids corporel selon l'une quelconque des revendications 1 à 4, dans lequel la seconde partie (142) est accouplée de manière articulée à la première partie (132).

6. Support de poids corporel selon l'une quelconque des revendications 1 à 5, dans lequel la longueur de la seconde partie (142) est réglable.

7. Support de poids corporel selon l'une quelconque des revendications 1 à 6, dans lequel la longueur de la première partie (132) est réglable.

8. Support de poids corporel selon l'une quelconque des revendications 1 à 7, dans lequel un angle entre la première partie (132) et la seconde partie (142) dans la première configuration du second élément est réglable.

9. Support de poids corporel selon l'une quelconque des revendications 1 à 8, comprenant en outre un accouplement pivotant configuré pour permettre la rotation du second élément par rapport au premier élément, le support de poids corporel comprenant en outre facultativement :
un élément formant levier accouplé au second élément, l'élément formant levier (270) étant configuré pour appliquer une force de frottement à l'accouplement pivotant pendant la rotation du second élément de la première position angulaire à la seconde position angulaire.

10. Support de poids corporel selon l'une quelconque des revendications 1 à 9, dans lequel l'élément porteur peut être désactivé pour permettre une rotation sensiblement libre du second élément par rapport au premier élément.

11. Support de poids corporel selon l'une quelconque des revendications 1 à 10, dans lequel le premier élément est configuré pour être placé contre une partie supérieure de jambe de l'utilisateur et le second élément est configuré pour être placé contre une partie inférieure de jambe de l'utilisateur, dans lequel le premier élément est configuré facultativement pour être placé contre le côté extérieur de la partie supérieure de jambe de l'utilisateur.

12. Support de poids corporel selon la revendication 11, dans lequel la seconde partie (142) peut être raccordée à une chaussure de l'utilisateur, dans lequel la seconde partie (142) peut être raccordée facultativement à l'arrière de la chaussure de l'utilisateur.

13. Support de poids corporel selon l'une quelconque des revendications 1 à 12, comprenant en outre un élément de raccordement (150) accouplé de manière pivotante à la première partie (132) du second élément, dans lequel l'élément de raccordement (150) vient en prise avec une extrémité de l'élément porteur, dans lequel la longueur de l'élément de raccordement (150) est éventuellement réglable.

14. Support de poids corporel selon la revendication 13, dans lequel une extrémité de l'élément de raccordement (150) est mobile de manière coulissante à l'intérieur d'une fente (116) dans le premier élément, et dans lequel l'extrémité de l'élément de raccordement (150) vient en prise avec l'extrémité de l'élément porteur.

15. Support de poids corporel selon l'une quelconque des revendications 1 à 12, dans lequel l'élément porteur comprend un câble (428, 528), dans lequel une première extrémité (438) du câble (428, 528) est éventuellement fixée à la seconde partie (142) du second élément.
